(19) Europäisches Patentamt / European Patent Office / Office européen des brevets

(11) **EP 4 067 448 A1**

(12) **EUROPEAN PATENT APPLICATION**

(43) Date of publication:
**05.10.2022 Bulletin 2022/40**

(21) Application number: **21166558.3**

(22) Date of filing: **01.04.2021**

(51) International Patent Classification (IPC):
***C09D 183/04*** *(2006.01)* ***C09D 183/08*** *(2006.01)*
***C08J 3/28*** *(2006.01)* ***C09D 5/08*** *(2006.01)*
***C09D 5/16*** *(2006.01)* ***B05D 3/06*** *(2006.01)*
***B05D 5/08*** *(2006.01)* ***B05D 7/14*** *(2006.01)*
***B08B 17/02*** *(2006.01)* ***B82Y 30/00*** *(2011.01)*
***B82Y 40/00*** *(2011.01)* *G02B 1/18 (2015.01)*
*C03C 17/30 (2006.01)* *C03C 23/00 (2006.01)*
*A61L 33/06 (2006.01)* *C08L 83/04 (2006.01)*
*A61B 1/313 (2006.01)* *A61B 1/12 (2006.01)*
*A61B 17/3211 (2006.01)* *C10M 107/50 (2006.01)*

(52) Cooperative Patent Classification (CPC):
(C-Sets available)
**C09D 5/1637; A61L 29/02; A61L 29/085; A61L 29/14; A61L 33/0088; B05D 3/065; B05D 5/08; C03C 17/30; C08J 3/28; C09D 5/00; C09D 5/008; C09D 5/08; C09D 5/1675; C09D 5/1681; C09D 5/1693;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Friedrich-Alexander-Universität Erlangen-Nürnberg**
**91054 Erlangen (DE)**

(72) Inventors:
- **TESLER, Alexander B.**
  **91054 Erlangen (DE)**
- **GOLDMANN, Wolfgang H.**
  **91054 Erlangen (DE)**
- **PRADO, Lucia H.**
  **91054 Erlangen (DE)**
- **FISCHER, Lena**
  **91054 Erlangen (DE)**
- **MAZARE, Anca**
  **91054 Erlangen (DE)**
- **VIRTANEN, Sannakaisa**
  **91054 Erlangen (DE)**
- **KHUSNIYAROV, Marat M.**
  **91054 Erlangen (DE)**
- **THIEVESSEN, Ingo**
  **91054 Erlangen (DE)**

(74) Representative: **Vossius & Partner**
**Patentanwälte Rechtsanwälte mbB**
**Siebertstraße 3**
**81675 München (DE)**

# (54) SILICONE- OR FLUOROSILICONE-COATED SOLID SUBSTRATES AND PROCESS FOR THEIR PREPARATION

(57) Provided is a process for the preparation of a silicone or fluorosilicone-coated solid substrate, which process comprises the following steps
a) providing a solid substrate having at least one surface to be coated,
b) bringing the surface to be coated in contact with a coating fluid comprising linear silicone or linear fluorosilicone molecules, and
c) irradiating the coating fluid in contact with the surface by UV light to photo-dissociate a silicon-carbon bond in linear silicone or linear fluorosilicone molecules contained in the coating fluid, and allowing them to form a new covalent bond to the surface of the solid substrate, thus providing a solid substrate having at least one surface that carries a coating comprising a layer of silicone or fluorosilicone molecules covalently grafted to the surface, and further comprising silicone or fluorosilicone molecules adhering non-covalently to the layer of the grafted molecules. Moreover, coated solid substrates are provided, which are obtainable by the process in accordance with the invention.

EP 4 067 448 A1

(52) Cooperative Patent Classification (CPC): (Cont.)
**C09D 183/04; G02B 1/18;** A61B 17/3211;
A61L 2420/02; B05D 2202/10; B08B 17/065;
B82Y 30/00; B82Y 40/00; C03C 2217/76

C-Sets
**A61L 29/085, C08L 83/04;**
**A61L 33/0088, C08L 83/04;**
**A61L 33/0088, C08L 83/06**

## Description

The present invention provides a process for the preparation of a silicone- or fluorosilicone-coated solid substrate and a coated solid substrate, which is obtainable by the process.

Surface wettability is one of the most important properties for various biological processes as well as in engineering and industrial applications. For instance, in biology, wetting/de-wetting controls the dynamics of adhesion on wet substrates such as mushroom spores and living cells. In industry, corrosion, which is a pure surface phenomenon occurs almost in all circumstances, where water-based liquids are in contact with solid material. Another vital example is the accumulation of unwanted material on solid surfaces that deteriorate their functionality by fouling. The fouling materials consist of either living organisms (*i.e.* biofouling) or non-living inorganic and/or organic compounds (*i.e.* chemofouling).

Several strategies have been developed to combat fouling. Inspired by the Lotus leaf architecture, superhydrophobic surfaces (SHS) emerged as a potential solution for creating self-cleaning antifouling surfaces (G. B. Hwang, K. Page, A. Patir, S. P. Nair, E. Allan, I. P. Parkin, ACS Nano 2018, 12, 6050). Moreover, slippery liquid-infused porous surfaces (SLIPS) have recently been introduced as an alternative approach to conventional SHS (A. Lafuma, D. Quéré, EPL (Europhysics Letters) 2011, 96, 56001; T.-S. Wong, S. H. Kang, S. K. Y. Tang, E. J. Smythe, B. D. Hatton, A. Grinthal, J. Aizenberg, Nature 2011, 477, 443). The slippery surface concept is based on the infusion of a porous substrate with a lubricating fluid that has a strong chemical affinity to the underlying substrate creating a stable, inert, and extremely smooth lubricant overlayer on the surface. There are several approaches to fabricate liquid-infused slippery surfaces. In general, SLIPS consists of three parts: (i) a structured rough substrate, (ii) an appropriate low surface energy coating, and (iii) a low-surface-tension fluid frequently called lubricant (R. Deng, T. Shen, H. Chen, J. Lu, H.-C. Yang, W. Li, J. Mat. Chem. A 2020, 8, 7536). Further examples of surfaces of this type are shown in WO 2012/100100 A2 and US 2019/0136070.

Silicone-based materials are widely used in everyday life and a broad range of industrial applications including medical technology, construction, automotive, food packaging, and paper industry. They are colorless, non-toxic, environmentally friendly, and biocompatible, have good thermal stability and excellent water repellency due to the organic-inorganic structure of siloxane molecules. Several approaches have been developed to graft silicones to solids. Polydimethylsiloxanes (PDMS), the most common silicone material, is usually grafted thermally on oxide surfaces (J. W. Krumpfer, T. J. McCarthy, Langmuir 2011, 27, 11514; S. T. Milner, T. A. Witten, M. E. Cates, Macromolecules 1988, 21, 2610; C.-H. Xue, X. Bai, S.-T. Jia, Sci. Rep. 2016, 6, 27262; L. Chen, S. Park, J. Yoo, H. Hwang, H. Kim, J. Lee, J. Hong, S. Wooh, Adv. Mater. Interfaces 2020, 7, 2000305; C. Zhang, Y. Xia, H. Zhang, N. S. Zacharia, ACS Appl. Mater. Interfaces 2018, 10, 5892). However, thermal grafting is a harsh, time-consuming, and expensive process not readily applicable to various materials. Recently, it was demonstrated that methyl-terminated PDMS can spontaneously bind to glass at room temperature, while the dissociation kinetics is extremely slow to form high-quality coatings (-100 h) (H. Teisala, P. Baumli, S. A. L. Weber, D. Vollmer, H.-J. Butt, Langmuir 2020, 36, 4416). An alternative approach is to apply silicone to a surface which comprises a metal-oxide photocatalyst to generate free radicals on the surface by UV light, which attacks the polymer molecules and allows them to form bonds with the surface (S. Wooh, N. Encinas, D. Vollmer, H.-J. Butt, Adv. Mater. 2017, 29, 1604637). Furthermore, PDMS was coated on surfaces by applying a silane-based adhesive (buffer) layer to the surface, or by mixing a crosslinking agent with PDMS (J. Wang, L. Wang, N. Sun, R. Tierney, H. Li, M. Corsetti, L. Williams, P. K. Wong, T.-S. Wong, Nat. Sustain. 2019, 2, 1097; C. Yang, F. Wang, W. Li, J. Ou, C. Li, A. Amirfazli, Appl. Phys. A: Mater. Sci. Process. 2015, 122, 1).

The invention provides a process for the preparation of a silicone- or fluorosilicone-coated solid substrate, which process comprises the following steps:

a) providing a solid substrate having at least one surface to be coated,
b) bringing the surface to be coated in contact with a coating fluid comprising linear silicone or linear fluorosilicone molecules, and
c) irradiating the coating fluid in contact with the surface by UV light to photo-dissociate a silicon-carbon bond in linear silicone or linear fluorosilicone molecules contained in the coating fluid, and allowing them to form a new covalent bond to the surface of the solid substrate, thus, providing a solid substrate having at least one surface that carries a coating comprising a layer of linear silicone or linear fluorosilicone molecules covalently grafted to the surface and further comprising linear silicone or linear fluorosilicone molecules adhering non-covalently to the layer of the grafted linear silicone or linear fluorosilicone molecules.

Moreover, silicone- or fluorosilicone-coated solid substrates are provided, which are obtainable by the process in accordance with the invention, and which have at least one surface that carries a coating comprising a layer of linear silicone or linear fluorosilicone molecules covalently grafted to the surface, and optionally linear silicone or linear fluorosilicone molecules adhering non-covalently to the layer of grafted silicone or fluorosilicone molecules.

The process of the present invention allows linear silicone or linear fluorosilicone molecules to be applied as coating on a variety of substrates such as metals, metal oxides, and ceramics.

A significant difference between the approach of the invention and grafting approaches described in the literature so far is that, in the process of the invention, silicone or fluorosilicone molecules are directly photo-activated, leading to a specific bond dissociation. Neither surface pre- or post-treatment, nor the presence of a photocatalyst are required, and the process can be carried out without any special equipment. The silicone or fluorosilicone molecules can serve both as surface energy reducing agent and lubricating oil, minimizing the interfacial energy between the solid substrate and lubricant. Surfaces of various dimensions and morphologies from smooth to rough, porous to non-porous can be coated, and hydrophobic and lubricant-infused slippery surfaces can be provided in a one-pot process. Further advantages of this approach are (i) the possibility of a simple and uniform implementation on a variety of materials and morphologies, (ii) rapid grafting kinetics, (iii) scalability, (iv) the possibility to use environmentally friendly, biocompatible materials, (v) the possibility of repeatable applications of the process on the same substrate, (vi) sterilizability, and (vii) low-cost.

Moreover, the silicone- or fluorosilicone-coated substrates which can be obtained by the process in accordance with the invention can be advantageously used in a variety of applications. The coated surfaces exhibit excellent corrosion protection for metals, repel complex liquids such as blood, reduce friction as well as substantially improve resistance to bacterial attachment when UV-grafted onto medically applicable substrates such as carbon- and stainless steel as well as glass lenses without deterioration of their mechanical and optical characteristics. Furthermore, the coated surfaces exhibit excellent repellent characteristics against the formation of biofilms from marine species.

The following items provide a summary of general and preferred aspects of the invention.

1. A process for the preparation of a silicone- or fluorosilicone-coated solid substrate, which process comprises the following steps:

    a) providing a solid substrate having at least one surface to be coated,
    b) bringing the surface to be coated in contact with a coating fluid comprising linear silicone or linear fluorosilicone molecules, and
    c) irradiating the coating fluid in contact with the surface by UV light to photo-dissociate a silicon-carbon bond in linear silicone or linear fluorosilicone molecules contained in the coating fluid, and allowing them to form a new covalent bond to the surface of the solid substrate, thus providing a solid substrate having at least one surface that carries a coating comprising a layer of linear silicone or linear fluorosilicone molecules covalently grafted to the surface and further comprising linear silicone or linear fluorosilicone molecules adhering non-covalently to the layer of the grafted linear silicone or linear fluorosilicone molecules.

2. The process in accordance with item 1, which further comprises, subsequent to step c), a step of removing excess coating fluid from the solid substrate.

3. The process in accordance with item 2, wherein excess coating fluid is allowed to flow off the solid substrate or is mechanically removed from the solid substrate.

4. The process in accordance with any of items 1 to 3, which further comprises, subsequent to step c), a step of removing linear silicone or linear fluorosilicone molecules non-covalently adhering to the layer of the grafted linear silicone or linear fluorosilicone molecules.

5. The process in accordance with any of items 1 to 4, wherein the surface to be coated comprises or consists of a material selected from metal, metal oxide, glass, ceramic, or silicon.

6. The process in accordance with any of items 1 to 5, wherein the surface to be coated comprises or consists of a material selected from steel, including stainless, martensitic or carbon steel, titanium, a titanium alloy, cobalt, magnesium, a magnesium alloy, glass, and porcelain.

7. The process in accordance with any of items 1 to 6, wherein the surface to be coated is free of a metal-oxide photocatalyst.

8. The process in accordance with any of items 1 to 7, wherein the solid substrate is selected from a medical device or a part thereof, a glass lens, marine equipment, a solar panel or a part thereof, and sanitary ware.

9. The process in accordance with any of items 1 to 8, wherein the coating fluid is free of any component that reacts

with the surface to be coated prior to or during steps b) or c), other than the linear silicone or linear fluorosilicone molecules.

10. The process in accordance with any of items 1 to 9, wherein the coating fluid is a neat silicone fluid or a neat fluorosilicone fluid.

11. The process in accordance with any of items 1 to 10, wherein the linear silicone or linear fluorosilicone molecules contain a trimethylsiloxy group as an end group at one or both ends; more preferably as an end group at both ends.

12. The process in accordance with any of items 1 to 11, wherein the linear silicone or linear fluorosilicone molecules are formed from identical or different repeating units of the following formula:

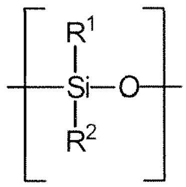

wherein $R^1$ and $R^2$ independently represent an optionally fluorinated C1-C6 alkyl group, preferably an optionally fluorinated C1-C3 alkyl group and most preferably a methyl group.

13. The process in accordance with any of items 1 to 12, wherein the coating fluid comprises or consists of polydimethylsiloxane.

14. The process in accordance with any of items 1 to 13, wherein the coating fluid has a viscosity at 25 °C of 2 to 1,000,000 $mm^2/s$, preferably 10 to 100,000 $mm^2/s$, and more preferably 20 to 10,000 $mm^2/s$.

15. The process in accordance with any of items 1 to 14, wherein bringing the surface to be coated in contact with the coating fluid involves immersing the solid substrate in the coating fluid or applying the coating fluid to the surface.

16. The process in accordance with any of items 1 to 15, wherein the irradiation of the coating fluid by UV-light in step c) is carried out for a period of 1 min or more, preferably 5 min or more.

17. The process in accordance with any of items 1 to 16, wherein the irradiation of the coating fluid by UV-light in step c) is carried out for a period 60 min or less, preferably 30 min or less.

18. The process in accordance with any of items 1 to 17, wherein the UV-light photo-dissociates a silicon-carbon bond in an end group of the linear silicone or linear fluorosilicone molecules.

19. The process in accordance with any of items 1 to 18, wherein the linear silicone or linear fluorosilicone molecules contain a trimethylsiloxy group as an end group at one or both ends, preferably as an end group at both ends, and wherein the irradiation by UV-light photo-dissociates a silicon-carbon bond in a trimethylsiloxy end group of the linear silicone or linear fluorosilicone molecules.

20. The process in accordance with any of items 1 to 19, wherein the irradiating UV light includes light at a wavelength of 321 nm or less.

21. The process in accordance with any of items 1 to 19, wherein the wavelength spectrum of the irradiating UV light has a peak of the spectral irradiance in the wavelength range of more than 317 to 321 nm.

22. The process in accordance with any of items 1 to 21, wherein the UV light used for the irradiation has a spectral irradiance for a wavelength in the range of more than 317 nm to 321 nm, which is higher than the spectral irradiance for each wavelength in the range of 317 nm and below.

23. The process in accordance with any of items 1 to 22, wherein step c) is accomplished while the coating fluid is kept at a temperature of less than 50 °C, preferably less than 30 °C, and more preferably at a temperature range of 15 to 25 °C.

24. A silicone- or fluorosilicone-coated solid substrate having at least one surface that carries a coating comprising a layer of linear silicone or linear fluorosilicone molecules covalently grafted to the surface, and optionally further comprising linear silicone or linear fluorosilicone molecules adhering non-covalently to the layer of the grafted linear silicone or linear fluorosilicone molecules, which is obtainable by the process in accordance with any of items 1 to 23.

25. The silicone or fluorosilicone-coated solid substrate in accordance with item 24, wherein the thickness of the layer of grafted linear silicone or linear fluorosilicone molecules, in the absence of linear silicone or linear fluorosilicone molecules which adhere non-covalently to the layer of the grafted linear silicone or Inear fluorosilicone molecules, is in the range of 3 to 100 nm, preferably at 5 to 20 nm.

26. The silicone or fluorosilicone-coated solid substrate in accordance with item 24 or 25, wherein the grafting density of the linear silicone or linear fluorosilicone molecules grafted to the coated surface is in the range of $6.0 \times 10^{16}$ to $4.0 \times 10^{18}$ molecules per $m^2$, preferably $1.0 \times 10^{17}$ to $4.0 \times 10^{18}$ molecules per $m^2$.

27. The silicone- or fluorosilicone-coated solid substrate in accordance with any of items 24 to 26, wherein the solid substrate is selected from a medical device or a part thereof, a glass lens, marine equipment, a solar panel or a part thereof, and sanitary ware.

28. Use of a silicone- or fluorosilicone-coated solid substrate in accordance with any of items 24 to 27 in an application requiring one or more characteristics selected from corrosion resistance, reduced surface friction, capability for self-cleaning, anti-chemo-fouling, anti-bacterial, anti-marine fouling, and blood-repellency.

In the following, the present invention will be described in further detail. It is understood that the following description supplements the above summary and *vice versa.* Moreover, as outlined in the above summary, aspects of the invention relate to a process for the preparation of a silicone- or fluorosilicone-coated substrate, a silicone or fluorosilicone-coated solid substrate that can be obtained by the process in accordance with the invention, and to uses of the silicone or fluorosilicone-coated solid substrate. Thus, it will be further understood that, unless specifically indicated to the contrary, information provided on general and preferred embodiments of the invention, e.g. with regard to suitable solid substrates, to silicones or fluorosilicones suitable for the coating, or to the composition and structure of the coating, will apply to all of these related aspects, even if it is not explicitly mentioned in the discussion of all of these aspects.

As a first step a), the process of the invention comprises the step a) of providing a solid substrate having at least one surface to be coated. On the surface to be coated, the silicone- or fluorosilicone-coating is formed when the process of the invention is carried out. In the process in accordance with the invention, steps a), b) and c) are carried out in the indicated order, i.e. step b) is carried out after step a), and step c) is carried out after step b).

Surfaces to be coated with a silicone or fluorosilicone coating in the context of the invention may, e.g., be surfaces for which at least one of the following characteristics is desired: corrosion resistance, reduced surface friction, capability for self-cleaning, anti-chemo-fouling, anti-bacterial, anti-marine fouling, and blood-repellency. As shall be explained below, such characteristics can be conveniently imparted to a surface of a solid substrate to which a silicone or fluorosilicone coating is applied in line with the present invention.

Due to the versatility of the process in accordance with the invention, the material of the surface to which the silicone or fluorosilicone coating is applied, is not particularly restricted. As examples, reference can be made to surfaces which comprise or consist of a material selected from a metal, a metal oxide, glass, ceramic, and silicon. As examples of a metal, reference can be made to Al, Cu, Co, Mo, Ti, or steel. Suitable types of steel include, e.g., stainless steel such as austenitic and martensitic stainless steels, or carbon steel. As an example of a metal oxide, reference can be made to aluminum oxide or to titanium oxide. As examples of glass, reference can be made to quartz glass, borosilicate glass, optical glass as it is used for the production of optical lenses, or to fluorine-doped tin oxide (FTO) glass.

Preferred examples are surfaces which comprise or consist of a material selected from steel, e.g. martensitic stainless steel or carbon steel, titanium (i.e. titanium in metallic form), a titanium alloy, cobalt, magnesium (i.e. magnesium in metallic form), a magnesium alloy, glass, silicon, and porcelain, or surfaces which comprise or consist of two or more of these preferred materials.

In accordance with the invention, the silicone or fluorosilicone coating can also be applied to composite surfaces comprising two or more different materials.

Depending on the type of solid substrate and the desired use thereof, the invention encompasses a process, wherein a coating is applied to the complete surface of the solid substrate, and a process, wherein a coating is applied only to selected regions of the surface. Examples of solid substrates on the surface of which a silicone or fluorosilicone coating can be advantageously provided in accordance with the invention are medically applicable substrates, e.g. a medical device or a part thereof, a glass lens, marine equipment, a solar panel or a part thereof, or sanitary ware.

Examples of a medical device encompass in particular devices which are contacted during their use with a

body fluid, e.g. blood, and/or which are inserted or implanted into a patient's body. As preferred examples of medical devices or parts thereof on which a silicone or fluorosilicone coating can be advantageously applied in line with the invention; reference can be made to a scalpel blade, and to medical devices comprising a glass lens, such as an endoscope or a laparoscope, or to a glass lens comprised by the device.

Examples of marine equipment encompass any substrate which comes into contact with freshwater or seawater during its use.

As noted above, one advantage of the invention is its versatility with regard to the type of surface on which the silicone or fluorosilicone coating is applied.

Thus, the surface to which the silicone or fluorosilicone coating is applied in accordance with the invention does not need to have any particular morphology, e.g. does not need to contain pores, which can be impregnated with silicone or fluorosilicone molecules. Likewise, the surface does not need to be pretreated in order to provide a specific surface morphology before the application of the silicone or fluorosilicone coating, such as a micro- or micro/nano-surface structuring. However, if this is desired in order to further enhance, e.g., the hydrophobic property of the obtained silicone or fluorosilicone coating, a conductive surface can be subjected to an electrochemical treatment prior to the application of the silicone or fluorosilicone coating.

Similarly, it is not necessary to chemically modify the surface to which the silicone or fluorosilicone coating is applied prior to contacting it with the coating fluid, e.g. it is not necessary to provide a primer layer between the surface of the solid substrate and the silicone or fluorosilicone coating, which primer layer comprises a crosslinking agent forming a bond to the material of the surface to be coated, and a bond to the silicone or fluorosilicone molecule. Rather, the linear silicone or fluorosilicone molecules are typically covalently grafted directly to the solid substrate, without any intervening layer or crosslinker being present between the surface of the solid substrate and the silicone or fluorosilicone coating.

It will be understood that the cleaning of the surface prior to contacting the surface to be coated with the coating fluid is not to be considered as a chemical modification of the surface, since a cleaning step merely removes impurities from the surface. Thus, e.g. step a) of the process of the present invention may involve cleaning the surface to be coated before it is contacted with the coating fluid.

As also noted above, the present invention provides versatility with regard to the material of the surface to be coated. For example, the surface does not have to consist of, or to comprise, a metal-oxide photocatalyst. $TiO_2$, ZnO, $SnO_2$, $CeO_2$, $Ag_2O$, $Fe_2O_3$, $WO_3$ and $V_2O_5$ are metal oxides known as photocatalysts. Thus, the surface to be coated preferably does not consist of $TiO_2$, ZnO, $SnO_2$, $CeO_2$, $Ag_2O$, $Fe_2O_3$, $WO_3$, or $V_2O_5$ or any combination thereof, more preferably the surface to be coated does not comprise any $TiO_2$, ZnO, $SnO_2$, $CeO_2$, $Ag_2O$, $Fe_2O_3$, $WO_3$ or $V_2O_5$. Generally, the surface to be coated does not need to contain any photo-catalytically active material.

In step b) of the process in accordance with the invention, the surface to be coated is brought into contact with a coating fluid comprising linear silicone or linear fluorosilicone molecules. Preferably, the coating fluid comprises the linear silicone molecules.

It will be understood that, unless indicated otherwise in a specific context, "or" is not used herein in an exclusive sense, such that the coating fluid comprising linear silicone or linear fluorosilicone molecules as referred to herein may comprise (i) linear silicone molecules, (ii) linear fluorosilicone molecules, but also (iii) linear silicone molecules and linear fluorosilicone molecules. Among these options, (i) and (iii) are preferred, and (i) is more preferred. For the sake of comprehensiveness, the linear silicone molecules and the linear fluorosilicone molecules are also collectively referred to herein as "linear (fluoro)silicone molecules". The same applies for other any other context, in which the term (fluoro)silicone is used herein, i.e. "(fluoro)silicone" collectively refers to silicone and fluorosilicone, and the silicone embodiment is the preferred embodiment.

As will be further understood by the skilled person, the linear (fluoro)silicone molecules comprise a linear (i.e. unbranched) chain of silicon and oxygen atoms, which are arranged in an alternating manner (e.g. illustrated as -Si-O-Si-O-Si- ...). In a linear silicone molecule, each of the silicon atoms except for the first and the last silicon atom in the chain further carries two hydrocarbon groups as substituents, e.g. groups selected from an alkyl group, an aryl group and an aralkyl group, preferably alkyl groups, and most preferably methyl groups. In a linear fluorosilicone molecule, each of the silicon atoms except for the first and the last silicon atom in the chain further carries two hydrocarbon groups, e.g. groups selected from an alkyl group, an aryl group and an aralkyl group, preferably alkyl groups, and it at least a part of the hydrocarbon groups one or more hydrogen atom is (are) replaced by a fluorine substituent. The end group which is present at each of the two chain termini of a linear (fluoro)silicone molecule can be illustrated by the formula $-O-SiR_3$. The groups R are typically alkyl groups, preferably methyl groups. These end groups thus provide the first and the last silicon atom in the chain of silicon and oxygen atoms of the (fluoro)silicone molecule.

Preferably, the linear (fluoro)silicone molecules comprised by the coating fluid are formed from identical or different repeating units of the following formula:

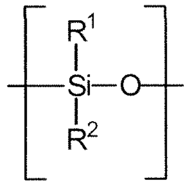

wherein $R^1$ and $R^2$ independently represent an optionally fluorinated C1-C6 alkyl group, preferably an optionally fluorinated C1-C3 alkyl group, and most preferably methyl. Thus, the linear (fluoro)silicone molecules are most preferably polydimethylsiloxane (PDMS) molecules.

As noted above, the linear (fluoro)silicone molecules have two end groups, one at each end. It is preferred that the molecules contain a trimethylsiloxy group as an end group at one or both ends, more preferably as an end group at both ends. Thus, a particularly preferred structure of the linear (fluoro)silicone molecules can be illustrated by the following formula:

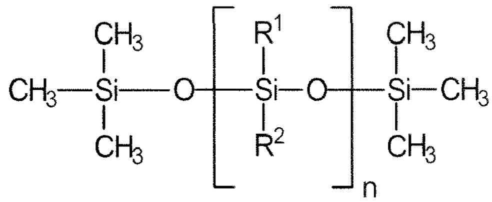

wherein $R^1$ and $R^2$ are defined as above, and n represents the degree of polymerization.

Most preferably, the linear (fluoro)silicone molecules comprised by the coating fluid are polydimethylsiloxane molecules having two trimethylsiloxy end groups.

In line with the above, a particularly preferred embodiment of the process in accordance with the present invention is a process for the preparation of a polydimethylsiloxane-coated solid substrate comprises the steps of a) providing a solid substrate having at least one surface to be coated, which surface comprises or consists of a material selected from steel, e.g. austenitic or martensitic stainless steel or carbon steel, titanium (i.e. titanium in metallic form), a titanium alloy, magnesium (i.e. magnesium in metallic form), a magnesium alloy, glass, silicon, and porcelain, or which comprises or consists of two or more of these preferred materials; b) bringing the surface to be coated in contact with neat polydimethylsiloxane having two trimethylsiloxy end groups as a coating fluid; and c) irradiating the coating fluid in contact with the surface by UV light to photo-dissociate a silicon-carbon bond in polydimethylsiloxane molecules contained in the coating fluid, preferably a silicon-carbon bond in an end group, and allowing them to form a new covalent bond to the surface of the solid substrate, thus providing a solid substrate having at least one surface that carries a polydimethylsiloxane coating comprising a layer of polydimethylsiloxane molecules covalently grafted to the surface and further comprising polydimethylsiloxane molecules adhering non-covalently to the layer of the grafted polydimethylsiloxane molecules. Also particularly preferred is a polydimethylsiloxane-coated solid substrate which is obtainable by this particularly preferred process variant.

As will be apparent from the above discussion, a linear (fluoro)silicone molecule comprises silicon-carbon bonds, i.e. covalent bonds between a silicon and a carbon atom.

The degree of polymerization of the linear (fluoro)silicone molecules is generally indicated as a number average degree of polymerization for the molecules under consideration, which can be calculated, e.g., from the number average molecular weight of the concerned (fluoro)silicone molecules determined by gel permeation chromatography (GPC).

For a linear silicone or linear fluorosilicone, the average degree of polymerization of the (fluoro)silicone molecules contained therein typically correlates with its viscosity, which is frequently indicated as a characterizing parameter. The viscosity (kinematic viscosity) at 25°C of the linear silicone or linear fluorosilicone which provides the (fluoro)silicone molecules comprised in the coating fluid is typically 2 $mm^2/s$ (cSt) or more, preferably 10 $mm^2/s$ or more, and more preferably of 20 $mm^2/s$ or more. The viscosity is typically 1,000,000 $mm^2/s$ or less, preferably 100,000 $mm^2/s$ or less, and more preferably 10,000 $mm^2/s$ or less. Thus, a typical range for the viscosity of the linear (fluoro)silicone is 2 to 1,000,000 $mm^2/s$, a preferred range is 10 to 100,000 $mm^2/s$, and a more preferred range is 20 to 10,000 $mm^2/s$.

While the above values for the viscosity are indicated for the linear (fluoro)silicone as such, the same typical and preferred upper and lower limits and ranges of the viscosity apply for the coating fluid, which is preferably a neat linear (fluoro)silicone fluid, i.e. a fluid which consists of linear (fluoro)silicone molecules. It is more preferably a neat silicon fluid which consists of linear silicone molecules, and it is most preferably neat polydimethylsiloxane which consists of the PDMS molecules. The viscosity (kinematic viscosity) at 25°C of the coating fluid is thus typically 2 $mm^2/s$ (cSt) or more, preferably 10 $mm^2/s$ or more, and still more preferably of 20 $mm^2/s$ or more. The viscosity is typically 1,000,000

$mm^2/s$ or less, preferably 100,000 $mm^2/s$ or less, and more preferably 10,000 $mm^2/s$ or less. Thus, a typical range for the viscosity of the linear (fluoro)silicone is 2 to 1,000,000 $mm^2/s$, a preferred range is 10 to 100,000 $mm^2/s$, and a more preferred range is 20 to 10,000 $mm^2/s$.

The viscosity (kinematic viscosity) of the (fluoro)silicone or the coating fluid comprising or consisting of the (fluoro)silicone at 25 °C can be determined, e.g., in accordance with ASTM D7042-21 or ASTM D4283-98(2015).

The number average molecular weight of the linear silicone or linear fluorosilicone which provides the (fluoro)silicone molecules comprised in the coating fluid is typically 0.4 kDa or more, preferably 1.3 kDa or more, and more preferably of 2.0 kDa or more. The number average molecular weight is typically 308 kDa or less, preferably 139 kDa or less, and more preferably 63 kDa or less. Thus, a typical range for the number average molecular weight of the linear (fluoro)silicone is 0.4 to 308 kDa, a preferred range is 1.3 to 139 kDa, and a more preferred range is 2.0 to 63 kDa. The number average molecular weight can be determined, e.g., by gel permeation chromatography.

In addition to the linear (fluoro)silicone molecules, the coating fluid may contain one or more further components, e.g. a diluent. However, such further components do not need to be contained therein. It is preferred that the coating fluid is free of any component which reacts with the surface to be coated prior to or during steps b) or c) of the process in accordance with the invention, other than the linear (fluoro)silicone molecules. For example, the coating fluid is typically free of a crosslinking agent forming a bond to the material of the surface to be coated and a bond to a linear (fluoro)silicone molecule. Photocatalytically active materials are also not required in the coating fluid and are typically absent from the coating fluid.

In the coating fluid, the linear (fluoro)silicone molecules generally provide the main component, so that more than 50 wt% of the total weight of the coating fluid, preferably 80 wt% or more, and more preferably 90 wt% or more of the total weight of the coating fluid are provided by the linear (fluoro)silicone molecules. Still more preferably, the coating liquid is a neat (fluoro)silicone fluid, i.e. a fluid which consists of one or more types of linear silicone molecules, or of one or more types of linear fluorosilicone molecules. Among them, the neat silicone fluid consisting of linear silicone molecules is again preferred. As will be understood from the above, the most preferred coating fluid is a neat polydimethylsiloxane silicon oil, wherein each polydimethylsiloxane molecule carries two trimethylsiloxy end groups.

The way in which the surface to be coated is brought into contact with the coating fluid is not particularly restricted. For example, the contact can be established by immersing the solid substrate, including the surface to be coated, in the coating fluid, or by applying the coating fluid to the surface. Methods for applying a fluid to a surface are well known, and include, e.g. pouring the fluid onto the surface, allowing the fluid to drip onto the surface, applying the fluid using a suitable tool, such as a brush, a roller, or a coating knife, or applying the fluid as a spray.

In step c), the coating fluid in contact with the surface is irradiated by UV light to photo-dissociate a silicon-carbon bond in linear silicone or linear fluorosilicone molecules contained in the coating fluid and allowing these molecules to form a new covalent bond to the surface of the solid substrate. Thus, a solid substrate is provided which has at least one surface (i.e. the surface in contact with the irradiated coating fluid) that carries a coating comprising a layer of linear silicone or linear fluorosilicone molecules covalently grafted to the surface. The surface that carries the coating is also referred to herein as coated surface.

Without wishing to be bound by theory, it is understood that the dissociation of a silicon-carbon bond, i.e. a covalent bond existing between a silicon atom and a carbon atom in a (fluoro)silicone molecule contained in the coating fluid, via irradiation by UV light generates a reactive (fluoro)silicone molecule in the form of a radical, which can form a new covalent bond with the surface that is in contact with the coating fluid. It is further understood that the new bond is generally formed between the silicon atom that formed part of the dissociated silicon-carbon bond and the surface. The formation of the new covalent bond can occur spontaneously when the reactive (fluoro)silicone molecule contacts the surface. This formation of a covalent bond between the reactive (fluoro)silicone molecule and the surface is also referred to herein as the reaction of the (fluoro)silicone molecule with the surface. The linear (fluoro)silicone molecules which have formed a covalent bond to the surface to be coated are referred to herein as (fluoro)silicone molecules which are grafted to the surface, or briefly as grafted (fluoro)silicone molecules.

Generally, not all of the (fluoro)silicone molecules contained in the coating fluid will react with the surface in step c), e.g. since the photodissociation of a silicon-carbon bond occurs in some, but not all of the molecules, or since some of the reactive (fluoro)silicone molecules are deactivated without reacting with the surface. Therefore, the coating carried by the solid substrate as it results from step c) generally comprises, in addition to the layer of the grafted linear (fluoro)silicone molecules, linear (fluoro)silicone molecules adhering non-covalently to the layer of the grafted linear (fluoro)silicone molecules. They can advantageously contribute to the hydro- or omniphobicity and/or to the lubricity of the coated surface.

The irradiation of the coating fluid by UV light in step c), also referred to as illumination, is carried out for a period of time which is sufficiently long to form a layer of grafted (fluoro)silicone molecules on the surface. It is typically carried out for a period of 1 min or more, preferably 5 min or more. As noted above, it is an advantage of the process that the formation of the coating layer proceeds rapidly. Thus, long irradiation times are possible, but not required. It has been found that the thickness of the layer of grafted (fluoro)silicone molecules initially increases with increasing irradiation

time, but that a maximum thickness can be achieved already within periods of less than an hour. Thus, for reasons of efficiency, the period for which the irradiation is carried out can be limited to 60 min or less, preferably 30 min or less.

Another advantage of the process in accordance with the present invention is that the photo-dissociation of bonds in the linear (fluoro)silicone molecules allows the activation of these molecules to be carried out in a controlled manner, and to avoid a non-specific dissociation of different bonds including Si-O bonds in the backbone of the (fluoro)silicone molecules. For example, if free radicals are generated via irradiation of a photocatalyst by UV light or via exposure to heat, the free radical could attack the (fluoro)silicone molecule to lead to non-specific oxidation or decomposition thereof.

By irradiating the linear (fluoro)silicone molecules in the coating fluid with UV light, it is possible to selectively dissociate a bond which does not form part of the silicon-oxygen backbone of the linear (fluoro)silicone molecule. This is due to the fact that the various types of bonds formed within the (fluoro)silicone molecules have different bond strengths or bond dissociation enthalpies. For example, the bond dissociation enthalpy of a silicon-oxygen bond is higher than the bond dissociation enthalpy of a silicon-carbon bond in the linear (fluoro)silicone molecules. This applies in a similar manner for bonds between the same types of atoms, e.g. between silicon and carbon, but with different binding partners attached to these atoms. For example, a silicon-carbon bond in a repeating unit of a polydimethylsiloxane molecule has a higher bond dissociation enthalpy compared to a silicon-carbon bond in trimethylsiloxy end group. Thus, by adapting the UV light accordingly, typically in terms of the wavelength used for irradiation, it is possible to photo-dissociate a bond in a defined position of a linear (fluoro)silicone molecule with a high selectivity. Thus, for example, the structural integrity of the grafted (fluoro)silicone molecules can be ensured, and an ordered structure of the grafted molecules can be achieved.

Thus, in the process of the present invention, it is preferred that the number of Si-O bonds in (fluoro)silicone molecules that is photo-dissociated during the irradiation by UV light in step c) is smaller, more preferably significantly smaller, than the number of Si-C bonds that is photo-dissociated. It is still more preferred that Si-O bonds are not photo-dissociated during the irradiation.

In the process of the present invention, it is also preferred that the UV-light used for the irradiation in step c) dissociates a silicon-carbon bond in an end group of the (fluoro)silicone molecules contained in the coating fluid. It is further preferred that the linear (fluoro)silicone molecules contain a trimethylsiloxy group as an end group at one or both ends, preferably as an end group at both ends, and that the irradiation by UV-light photo-dissociates a silicon-carbon bond in a trimethylsiloxy end group of the linear silicone or linear fluorosilicone molecules. It is further preferred that the number of Si-C bonds in repeating units of (fluoro)silicone molecules that is photo-dissociated during the irradiation by UV light in step c) is smaller, more preferably significantly smaller, than the number of Si-C bonds in end groups that is photo-dissociated.

In order to effectively photo-dissociate silicon carbon bonds in the (fluoro)silicone molecules, the UV light used for the irradiation in step c) typically includes UV light with a wavelength of 321 nm or less. With a view to the selectivity of the photo-dissociation, it is preferred that the UV light used for the irradiation in step c) includes UV light with a wavelength of more than 300 nm to 321 nm, and it is more preferred that the UV light used for the irradiation in step c) includes UV light with a wavelength of more than 317 nm to 321 nm. For example, UV light can be advantageously be used with a wavelength spectrum which has a peak of the spectral irradiance in the wavelength range of more than 317 to 321 nm (indicated in terms of the wavelength of the peak maximum).

With a view to the selectivity of the photo-dissociation, it is further preferred for the UV light used for the irradiation that the spectral irradiance for a wavelength in the range of more than 300 nm to 321 nm is higher than the spectral irradiance for each wavelength in the range of 300 nm and less, or that the UV light does not comprise light with a wavelength of 300 nm and less. It is still further preferred for the UV light used for the irradiation that the spectral irradiance for a wavelength in the range of more than 317 nm to 321 nm is higher than the spectral irradiance for each wavelength in the range of 317 nm and less, or that the UV light does not comprise light with a wavelength of 317 nm and less.

Generally, the photo-dissociation of silicon-oxygen bonds can be minimized or avoided by minimizing or avoiding the irradiation with UV light having a wavelength of 220 nm or less.

As will be understood by the skilled reader, a UV light source with a suitable wavelength range can be selected for carrying out the process in accordance with the invention, where desired in combination with a wavelength filter excluding certain wavelength ranges from the light used for irradiation. As an example of a suitable UV light source for use in the process in accordance with the invention, a mercury lamp, e.g. a medium pressure mercury lamp, fluorescent lamp, or a UV-LED, e.g. a UVA-LED, can be mentioned.

In order to provide a (fluoro)silicone coating in accordance with the present invention, neither the solid substrate or its surface, nor the coating fluid needs to be heated. Thus, step c) can be conveniently accomplished while the coating fluid is kept at a temperature of less than 50 °C, preferably less than 30 °C, and more preferably at a temperature in the range of 15 to 25 °C.

As noted above, generally not all of the (fluoro)silicone molecules contained in the coating fluid will be grafted to the surface in step c). Therefore, the coating carried by the solid substrate as it results from step c) generally comprises

the layer of the grafted linear (fluoro)silicone molecules and comprises in addition linear (fluoro)silicone molecules adhering non-covalently to the layer of the grafted linear (fluoro)silicone molecules. For example, these molecules can adhere via entanglement of the (fluoro)silicone chains to the grafted molecules. Such a coating comprising linear (fluoro)silicone molecules adhering non-covalently to the layer of the grafted linear (fluoro)silicone molecules is also referred to herein as a lubricant-infused coating, since the additional (fluoro)silicone molecules can advantageously contribute to the hydro- or omniphobicity and/or to the lubricity of the coated surface.

The process in accordance with the invention allows the preparation of such a lubricant-infused coating in a one-pot-process, i.e. the layer of grafted (fluoro)silicone molecules and the infusing lubricant can be applied in a single step.

The process of the present invention may further comprise, after step c), a step of removing excess coating fluid from the solid substrate, e.g. by allowing the excess coating fluid to flow off the substrate, or by mechanically removing it from the substrate, for example by wiping it off.

Although the (fluoro)silicone molecules adhering non-covalently to the layer of the grafted linear (fluoro)silicone molecules do not form a covalent bond to the surface, they adhere rather stably to the surface and can typically not easily be mechanically removed. Thus, if excess coating fluid is removed subsequent to step c), the linear (fluoro)silicone molecules acting as infusing lubricant are typically retained in the coating. However, these (fluoro)silicone molecules can be removed, if desired, e.g. in order to obtain a coating comprising or consisting only of grafted linear (fluoro)silicone molecules, e.g. in cases where an elution of (fluoro)silicone molecules from the coating over time should be avoided.

The process of the invention may comprise, after step c), a step of removing (fluoro)silicone molecules non-covalently adhering to the layer of grafted (fluoro)silicone molecules. This can be accomplished, e.g., by thoroughly rinsing the coating with a solvent for the linear (fluoro)silicone molecules. If necessary, the rinsing of the coating can be repeated one or more times.

Thus, as noted above, the process of the present invention can provide a silicone- or fluorosilicone-coated solid substrate having at least one surface that carries a coating comprising a layer of linear silicone or linear fluorosilicone molecules covalently grafted to the surface, and further comprising linear silicone or linear fluorosilicone molecules adhering non-covalently to the layer of the grafted linear silicone or linear fluorosilicone molecules (also referred to as a lubricant-infused coating). It can also provide a silicone- or fluorosilicone-coated solid substrate having at least one surface that carries a coating comprising a layer of linear silicone or linear fluorosilicone molecules covalently grafted to the surface, and which does not comprise linear silicone or linear fluorosilicone molecules adhering non-covalently to the layer of the grafted linear silicone or linear fluorosilicone molecules, e.g. if the linear silicone or linear fluorosilicone molecules adhering non-covalently to the layer of the grafted linear silicone or linear fluorosilicone molecules are removed from the coating originally resulting from step c) of the process in accordance with the invention. Therefore, the linear silicone or linear fluorosilicone molecules adhering non-covalently to the layer of the grafted linear silicone or linear fluorosilicone molecules represent an optional component of the coating carried by the coated solid substrate provided by the present invention.

In the silicone or fluorosilicone-coated solid substrate obtainable by the process of the present invention, the thickness of the layer of grafted linear silicone or fluorosilicone molecules in the absence of the linear silicone or fluorosilicone molecules adhering non-covalently to the layer of grafted (fluoro)silicone molecules can be determined, e.g., via ellipsometry. The thickness is preferably 3 nm or more, more preferably 5 nm or more, and still more preferably 7 nm or more. It is typically 100 nm or less, preferably 20 nm or less. Generally, (fluoro)silicones with a higher molecular weight or a higher viscosity yield thicker layers. Similarly, longer irradiation times may increase the layer thickness to a certain extent.

In the silicone or fluorosilicone-coated solid substrate obtainable by the process of the present invention, the grafting density $\sigma$ of the grafted linear silicone or fluorosilicone molecules in the absence of the linear silicone or fluorosilicone molecules adhering non-covalently to the layer of grafted (fluoro)silicone molecules can be calculated using the formula

$$\sigma = (H\rho N_A)/M_w$$

wherein, H is the slope of a straight line fitted to the datapoints in a log-log plot of the thickness of the layer of grafted linear silicone or fluorosilicone molecules, e.g. as determined via ellipsometry as a function of the number average molecular weight of the (fluoro)silicone, e.g. as determined via GPC; $\rho$ is the bulk density of the (fluoro)silicone; $N_A$ is Avogadro's number; and $M_w$ is the number average molecular weight. The grafting density calculated in this manner can be e.g. in the range of $6.0 \times 10^{16}$ to $4.0 \times 10^{18}$ molecules per $m^2$. Preferably, it is in the range of $1.0 \times 10^{17}$ to $4.0 \times 10^{18}$ molecules per $m^2$. Denser layers are typically formed by (fluoro)silicones with a lower molecular weight or a lower viscosity.

As noted above, the silicone or fluorosilicone-coated solid substrate provided by the present invention are

suitable for use in an application requiring one or more characteristics selected from corrosion resistance, reduced surface friction, capability for self-cleaning, anti-chemo-fouling, anti-bacterial, anti-marine fouling, and blood-repellency.

As noted above, preferred examples of solid substrates on the surface of which a silicone or fluorosilicone coating can be advantageously provided in accordance with the invention are medically applicable substrates, e.g. a medical device or a part thereof, a glass lens, marine equipment, a solar panel or a part thereof, or sanitary ware.

For example, the (fluoro)silicone coatings provided by the present invention can be advantageously applied to solar panels to employ self-cleaning characteristics of slippery lubricant-infused surfaces. A coated solar cell surface can be cleaned passively by rain, restoring its efficiency.

Due to the biocompatibility of silicone-based compounds, the coatings can also be advantageously applied on medically applicable substrates, such as scalpel blades and lenses, that display enhanced corrosion resistance, reduced friction through an incision, and repel blood staining and bacterial adhesion without deteriorating their mechanical and optical characteristics. There are several advantages when this technology is used to provide (fluoro)silicone coatings on a medical equipment or medical device surface:

- No harsh surface treatments are required to modify the bare surfaces of a device.
- The treated surfaces can be sterilized before and/or after any medical treatment.
- The process of applying the (fluoro)silicone coating can be applied virtually an infinite number of times.
- A sterilization can be accomplished via UV irradiation simultaneously with the grafting of (fluoro)silicone molecules to the surface.
- The optical field of view can be extended in minimally-invasive surgical devices passively and without the need for any external energy/stimuli.
- The grafting kinetics is rapid and the coating process can be accomplished within a short time, which makes it suitable for the continuous use on surgical instruments in hospitals.

Thus, for example, a coated medical device surface provided in accordance with the invention can be mechanically wiped and/or chemically cleaned after a medical treatment, then re-grafted using the process in accordance with the invention and simultaneously sterilizing it by irradiation with UV-light.

Due to biocompatibility, a coating can be applied in accordance with the invention on implantable surfaces for humans and animals, e.g. implants comprising or consisting of a titanium alloy, a magnesium alloy, or stainless steel.

A reduction of the friction coefficient which can be achieved by the application of the coatings in accordance with the invention can be advantageous, e.g., for scalpel blades used in robotically driven surgeries since an incision made by coated scalpel blades is more precise and needs less pressure to cut.

Furthermore, coated metal surfaces provided in accordance with the invention can contribute to the corrosion inhibition of the metals.

The coatings provided by the present invention are also suitable to repel marine fouling species such as algae and diatoms. A significant reduction in the biofilm formation coated substrates both of the lubricant-infused type and on coated substrates not comprising infusing lubricant could be achieved in comparison to bare analogs.

Due to the simple implementation, the coatings provided in accordance with the invention can be applied to sanitation components to substantially reduce the amount of fresh water spent, e.g. in toilets.

In line with the above, particularly preferred examples of coated solid substrates provided by the present invention are selected from a polydimethylsiloxane coated solid substrate selected from a medical device or a part thereof, a glass lens, marine equipment, a solar panel or a part thereof, and sanitary ware, which solid substrate carries on at least one surface thereof a coating comprising a layer of polydimethylsiloxane molecules covalently grafted to the surface, and which optionally comprises in addition polydimethylsiloxane molecules adhering non-covalently to the layer of the grafted polydimethylsiloxane molecules. Also in this context, as preferred examples of medical devices or parts thereof on which a PDMS coating can be advantageously applied in line with the invention, reference can be made to a scalpel blade, and to medical devices comprising a glass lens, such as an endoscope or a laparoscope, or to a glass lens comprised by the device.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The following examples further illustrate the invention.

## Examples

### 1. Materials and Methods

*Materials:* 1×3 inch microscope glass slides (VWR, Germany), coverslips (#4, Menzel-Glaser, Germany), quartz slides (1-mm-thick, GVB, Germany), p-type Si wafer ($\mu$Chemicals, Germany), $SiO_2$/Si wafer (3 in. Si(100) p-type with 100 nm $SiO_2$, $\mu$Chemicals, Germany), 0.5-mm-thick Co foil (99.9 %, Advent Research Materials, UK), 1-mm-thick Al sheets (99.95 %, Advent Research Materials, UK), 0.2-mm-thickness Mo foil (99.95 %, Advent Research Materials, UK), 0.5-mm-thick Ti foil (99.99 %, Advent Research Materials, UK), 0.6-mm-thick Cu foil (Marawe, Germany), carbon steel (Bayha, Germany), and martensitic stainless steel with the following nominal composition (wt.%): 0.6 to 0.7% C, 12% Cr, ≤ 0.5% Ni, ≤ 0.5% Si, ≤ 1% Mn, ≤ 0.03% P, and ≤ 0.025% S (Swann-Morton, UK) surgical blades #22 were used as substrates. Before surface functionalization, the substrates were cleaned ultrasonically in acetone and ethanol for 10 min and then dried under a stream of $N_2$. Silicone oil of 2, 100, 500, 1000, 100000, and 500000 cSt ($M_w$ = 0.41, 6, 17.3, 28, -140, and -190 kDa, respectively), toluene, ethanol, and acetone were purchased from Carl Roth, Germany, and used as received.

*Stainless steel and Ti anodization:* The electrochemical anodization was performed in a standard two-electrode-cell using LAB/SM 7300 DC power supply (ET System, Germany), 316L grade stainless steel (Advent Research Materials) as an anode, and Pt foil as a cathode. Stainless steel samples were anodized in a two-step procedure as previously reported (L. Prado, E. Anastasiou, S. Virtanen, J. Electrochem. Soc. 2021, 168, 021507). Briefly, an ethylene glycol solution containing 0.1 M of $NH_4F$ and 0.2 M $H_2O$ was used as an electrolyte, and the anodization was performed at 20 °C and at an applied potential of 90 V. The duration of the first step anodization was 20 min. Then, the porous anodic layer was removed using an UP100H ultrasonic processor (Hielscher, Germany) in water for 5 min and dried by a stream of $N_2$. Thereafter, a further anodization step was performed for 10 min. After the second-step anodization, the samples were extensively rinsed with a 20:80 (v/v) water-ethanol mixture and then kept in ethanol until annealed in air at 400°C for 1 h. Ti foil was anodized according to our previously published procedure (A. B. Tesler, M. Altomare, P. Schmuki, ACS Appl. Nano Mater. 2020, 3, 10646). Briefly, the Ti foil was anodized in a molten o-$H_3PO_4$ electrolyte containing 3 M of $NH_4F$ for 120 min at an applied potential of 15 V and 100 °C. The "as-anodized" samples were immersed in DI water for 12 h to remove residual electrolytes and finally dried under a stream of $N_2$. Well-defined, self-organized Ti nanotubular arrays with a thickness of ~200 nm were obtained.

*Al foil oxidation:* Al sheets were oxidized in boiling water for 10 min to form a rough nanometer-scale pseudoboehmite aluminum oxide layer and then dried under a stream of $N_2$ (A. B. Tesler, T. Sannomiya, A. Vaskevich, E. Sabatani, I. Rubinstein, Adv. Opt. Mater. 2018, 6, 1800599).

*Surface functionalization and lubrication:* The samples were horizontally placed in a Petri dish with a quartz cover. Approximately 20 $\mu$L $cm^{-2}$ of silicone oil (PDMS) was then dropped to cover the entire sample surface and uniform coverage was achieved by tilting the sample. The samples were illuminated by UV (Hg) lamp at 400 W (Noblelight DQ2523, Heraeus, Germany). The emission maxima of this lamp are in the UV range, *i.e.* $\lambda$ = 320 and 370 nm. The working distance between the lamp and the sample was 15 cm. The UV light power density was measured using a 1830-C Newport optical power meter equipped with a 818-UV/DB optical power detector and 1 % Newport ND filter. The power density at the working distance is about 50 mW $cm^{-2}$. To measure the water contact angle and hysteresis on UV-grafted PDMS layers, the remnant oil was dissolved by extensive rinsing in toluene, and then dried under a stream of $N_2$.

*Scalpel blade and glass lens functionalization:* To form lubricant-infused slippery scalpels, sterile medical scalpel blades #22 made of (i) carbon steel (Bayha, Germany) and (ii) martensitic stainless steel (Swann-Morton, UK) were placed horizontally in a glass Petri dish with a quartz cover. The silicon oil was dropped on the blade surface and then the samples were illuminated by a UV lamp for 30 min. The procedure was repeated on every scalpel side. To form UV-grafted PDMS surfaces, the silicone oil was dissolved by an extensive rinse in toluene. The same procedure was also applied on soda-lime, double convex glass optical lenses of 50 mm in diameter.

*Morphology and physicochemical characterization:* For morphological characterization, a field-emission scanning electron microscope (Hitachi FE-SEM S4800) was used equipped with energy-dispersive X-ray spectroscopy (EDAX, Genesis). The composition and the chemical state of the films were characterized using X-ray photoelectron spectroscopy (XPS, PHI 5600, US), and the spectra were shifted according to the C 1s signal at 284.8 eV, and the peaks were fitted by MultiPak software. Depth profiling was carried out using the instrument's $Ar^+$ sputter source operated at 3 kV and 15 nA, rastered over a 3 × 3 $mm^2$ area at a sputtering angle of 45° to the surface normal. Sputter steps of 1 min were repeated till the Si substrate. The atomic composition was determined between consecutive sputtering intervals, by evaluating the photoelectron peak area, using MultiPak processing software. The sputtering rate was calibrated using commercial Si/$SiO_2$ wafers (3 in. Si(100) p-type with 100 nm $SiO_2$, $\mu$Chemicals, Germany) and found as 2 nm $min^{-1}$.

*ToF-SIMS measurements:* Positive and negative static SIMS measurements were performed on a ToF-SIMS 5 spectrometer (ION-TOF, Münster). The samples were irradiated by a pulsed 25 keV $Bi_3^+$ liquid-metal ion beam. Spectra

were recorded in high mass-resolution mode (m/Δm > 8000 at $^{29}Si$). The beam was electrodynamically bunched down to 25 ns to increase the mass resolution and raster-scanned over a 125 × 125 $\mu m^2$ area. The primary ion dose density (PIDD) was kept at ~5 × $10^{11}$ ions × $cm^{-2}$ ensuring static conditions. Signals were identified using the accurate mass as well as their isotopic pattern and profile. Negative depth profiles are recorded in dual beam mode with a pulsed 25 keV $Bi^+$ liquid-metal ion beam (bunched down to <0.8 ns) for spectra generation and a 500 eV $Cs^+$ ion beam for sputter-removal on a 50 × 50 $\mu m^2$ area in the center of the 300 × 300 $\mu m^2$ sputter crater. Signals are identified according to their isotopic pattern as well as to their exact mass. Spectra are calibrated to $CH_2^-$, $C_2^-$ (negative polarity) and $C^+$, $CH^+$, $CH_2^+$, $CH_3^+$, and $C_7H_7^+$ (positive polarity) and a Poisson correction is used.

*Contact angle measurements:* The contact-angle measurements were performed by a contact angle measurement system (DSA100, Kruss, Germany) at room temperature. The droplet volume for the measurements was 10 $\mu$L, unless otherwise specified, and the macroscopic droplet profile was captured on camera. The droplet profile was fitted by a Drop Shape Analysis computer program provided by the manufacturer. Contact-angle hysteresis was measured by increasing and decreasing the droplet volume and video recording to determine the advancing and receding contact angles. All contact angle values specified in the text were averaged by at least 3 independent measurements.

*Ellipsometric measurements:* The thickness of PDMS layers was determined with a phase-modulated ellipsometer equipped with a 633 nm He-Ne laser source (Picometer Ellipsometer; Beaglehole Instruments, New Zealand). To achieve sufficient sensitivity for the detection of thin silicone layers, we performed angle scans around the Brewster angle of Si (i.e. 76°) (J. Colter Stewart, M. N. Shelley, N. R. Schwartz, S. K. King, D. W. Boyce, J. W. Erikson, D. D. Allred, J. S. Colton, Opt. Mater. Express 2019, 9, 4677) from 70° to 80° at a step width of 1° at three different positions of the sample surface. Angle-resolved data from ellipsometry were fitted assuming a 4-layer model by refractive indices of $n_{air}$ = 1.00, $n_{PDMS}$ = 1.403, $n_{SiO2}$ = 1.457, $n_{Si}$ = 3.4, and $k_{Si}$ = 0.03 for air, PDMS layers, native silicon oxide, and silicon wafer, respectively, while a plane Si wafer (with native oxide layer) was used as reference.

*Corrosion measurements:* The electrochemical polarization curves were measured in a standard three-electrode-cell using an IM6 electrochemical workstation (Zahner-Elektrik, Germany) and analyzed by the instrument's electrochemical software. Bare stainless steel, anodized stainless steel with grafted PDMS layer (superhydrophobic), anodized slippery stainless steel with grafted, and lubricated PDMS were used as working electrodes. Besides, pure copper and pure aluminum substrates were analyzed with bare, UV-grafted, and infused by PDMS oil of 500 cSt viscosity. The electrochemical tests were carried out in a standard 3.5 wt.% NaCl aqueous solution as corrosion medium. The cathode was a Pt electrode (20 × 20 mm), the reference electrode was an Ag/AgCl leakless (3.5 M KCl) reference electrode (ET072 eDAQ), and the scanning rate was fixed to 3 mV/s. The potentiodynamic polarization curves were started at -300 mV *vs.* the open circuit potential (OCP). In the anodic direction, the polarizations were performed up to 1.6 V for the AISI 316L grade stainless steel; 1.2 V for the pure Al, and 1.0 V for the pure Cu substrates. The calculation of the percentage inhibition efficiency (*IE*) of the corrosion current was performed as follows: $IE = ((i_0 - i_c)/i_0) \times 100\%$, where $i_0$ is the corrosion current of the bare sample, and $i_c$ is the corrosion current of the coated sample (UV-grafted or lubricant-infused). These values were obtained by the Tafel method after performing the polarization curves.

*Blood attachment experiments:* Fresh pig blood was generously provided by Unifleisch GmbH (Erlangen, Germany). Sodium citrate (0.5 vol.%) was added to the blood before receipt (at the lab facility) to prevent blood clotting. It was rotated on a mixer at 20 rpm to homogenize the contents before sampling.

*Bacterial Stock Preparation:* The wild-type bacterial strain *Escherichia coli* (*E. coli*) DH 5alpha strain and *Staphylococcus aureus* (*S. aureus*) were used. The stock culture was generated from an agar stock plate, from which one or two colonies were transferred to the growth medium. All cultures were incubated overnight at 37 °C in 5 mL of Tryptic Soy Broth growth medium (Becton Dickinson, Heidelberg) and kept shaking continuously in an orbital shaker at a speed of 150 turns $min^{-1}$. The concentration of bacteria was measured by UV-Vis spectroscopy at a wavelength of 600 nm wavelength ($OD_{600nm}$) *vs.* pure medium (control) and calculated according to the equation: 1.000 $OD_{600nm}$ = 8 × $10^8$ CFU $mL^{-1}$ (J. A. Myers, B. S. Curtis, W. R. Curtis, BMC Biophysics 2013, 6, 4).

*Bacterial biofilm adhesion:* Bare polished, anodized (hydrophilic), PDMS UV-grafted (hydrophobic), and slippery (lubricant-infused) stainless steel samples were placed horizontally in a 10 cm petri dish with 3 samples of 2 $cm^2$ per dish. The *E. coli* and S. *aureus* stock solution was diluted to $OD_{600nm}$ = 0.55 by growth medium. Thereafter, the surface of each sample was covered with 900 $\mu$L of the bacteria solution. Immediately following inoculation, all samples were incubated at 37 °C for 24 h to allow proper biofilm growth. Triplicates were done for both (*E. coli* and *S. aureus*) infused samples and non-infused controls. Biofilm coverage was analyzed by staining all samples in Neisser's solution II (Crystal violet, Carl Roth. Germany). This was followed by gentle rinsing in DI water to remove excess stain and then air-dried. Images were captured by confocal microscopy (Leica, Wetzlar), bright-field optical microscopy (Nikon, Japan), and SEM microscopy (Hitachi, Japan, without any additional pretreatment using a reduced working voltage).

*Bacterial adhesion to medical scalpel blades:* Bacteria adhesion was achieved by dipping untreated or treated

scalpel blades into a stock solution containing *E. coli* or *S. aureus* at $OD_{600nm}$ = ~1.000 in Tryptic Soy Broth culture medium. The scalpel blades were first kept submerged for 1 min in the *E. coli* or *S. aureus* solution then withdrawn and rinsed in 4 mL of the culture medium. The washing solution was measured by UV-vis spectroscopy at $\lambda$ = 600 nm. After the wash, the scalpels were used to scissor the agar in the petri dishes, which were then incubated for 24 h at 37 °C. The scalpel blades were sterilized by a brief rinse in 70 % ethanol aqueous solution between the subsequent submersions in different bacterial cultures. Three replicates per treatment were used. Statistical analysis (two-tailed t-test) was performed to determine the statistical significance of bacterial resistance.

*Marine biofoulers adhesion to steel substrates:* Freshwater green microalgae (Chlamidontas reinhardtii, Chlorella sorokiniana) were purchased from Culture Collection of Algae and Protozoa (CCAP, The Scottish Association for Marine Science). Freshwater algae were cultured in a 3N-BBM+V medium dissolved in DI water. The growing solution was measured by UV-vis spectroscopy at $\lambda$ = 600 nm. The steel samples were used as a substrate. As-produced, polished, UV-grafted and lubricant-infused surfaces were examined by immersion in marine culture solutions for 8 days under static conditions. The daylight cycle was kept 16 hours on and 8 hours off. The samples have been then removed from the growing medium solution without any other cleaning processes and allowed to dry in an ambient atmosphere. Bare glass slides were used as control samples. The growing solution was measured by UV-vis spectroscopy at $\lambda$ = 600 nm after 8 days of sample immersion.

*Theoretical calculations:* The program ORCA 4.1.1 was used for all calculations (Neese, F. ORCA 4.1.1 - Ab Initio, DFT and semiempirical electronic structure package. Max-Planck-Institut für Kohlenforschung, Mülheim/Ruhr, Germany). Geometry optimizations were performed on a truncated model for PDMS: $Me_3Si$-O-$SiMe_2$-O-$SiMe_3$. The bond of interest was homolytically broken, and the two resulting radical fragments (S = 1/2 each) were independently optimized. The true minima were confirmed by calculating vibrational frequencies showing no negative values. M06-2X functional (Y. Zhao, D. G. Truhlar, Theor. Chem. Acc. 2008, 120, 215) and def2-TZVP basis set (F. Weigend, R. Ahlrichs, Phys. Chem. Chem. Phys. 2005, 7, 3297) for all atoms were used. To speed up the calculations, RIJCOSX approximation with def2/J auxiliary basis sets (F. Weigend, Phys. Chem. Chem. Phys. 2006, 8, 1057) were employed. When calculating bond-dissociation enthalpy $DH^0_{293}$, zero-point energy and thermal energy corrections were applied routinely.

## 2. Results and discussion

### 2.1. Physicochemical characterization of UV-grafted PDMS layers.

**Figure 1a** shows the schematic representation of the UV-grafted PDMS-infused slippery surface formation process. The coating was first applied on bare Si wafers. The substrates were cleaned ultrasonically in acetone and ethanol and then dried under a stream of $N_2$. These samples display the water contact angle (WCA) of 48° $\pm$ 1° (Figure 1b). The cleaned samples were then placed horizontally on a Pyrex petri dish and a thin layer of silicone oil was dropped on the substrates allowing it to spread over the entire sample surface. The samples were illuminated by UV light at various time points from 5 to 60 min resulting in the formation of PDMS-infused slippery surfaces. To study the UV-grafted PDMS layers, the remnant oil was removed by an extensive rinse in toluene (S. Wooh, N. Encinas, D. Vollmer, H.-J. Butt, Adv. Mater. 2017, 29, 1604637, followed by a wash in ethanol, and dried under a stream of $N_2$. Since silicone oil is a sticky substance, the remnant oil, as well as unbound molecules, should be properly washed away. The stains of excessive oil can easily be observed on flat Si and Si/$SiO_2$ wafers by the appearance of a thin-film interference pattern, while on the other samples, *i.e.* polished metals and transparent glasses, cannot be easily detected. The UV-grafting reaction yielded a 6 - 9 nm thick coating as a function of UV illumination time on a flat Si wafer by ellipsometry measurements using 500 cSt PDMS oil (Figure 1c). As shown, the plain Si wafer is hydrophilic due to the native oxide with a thickness of ~1.82 $\pm$ 0.03 nm correlating well with the literature. Once UV-grafted and rinsed in toluene, Si wafers turn hydrophobic displaying the water contact angle (WCA) of 106° $\pm$ 1° and a contact angle hysteresis (CAH) of ~10° after only 5 min of UV light irradiation (Figure 1b). Between 5 and 60 min of the illumination, WCA does not change significantly, while CAH is slightly decreasing (Figure 1c). The WCA values achieved in this study are in agreement with reported values on flat substrates (L. Chen, S. Park, J. Yoo, H. Hwang, H. Kim, J. Lee, J. Hong, S. Wooh, Adv. Mater. Interfaces 2020, 7, 2000305). The overall thickness of the grafted PDMS layer increased steadily up to 30 min of illumination then remained constant well within the standard deviation. This trend was also confirmed by EDX measurements carried out on UV-grafted PDMS layers on anodized stainless steel (cf. Figure 1d and Table 1 below). The latter results may be attributed to the higher order (formation of a denser layer) of the grafted PDMS molecules rather than to the linear growth of attached molecules as previously shown by water-assisted PDMS-grafting (H. Teisala, P. Baumli, S. A. L. Weber, D. Vollmer, H.-J. Butt, Langmuir 2020, 36, 4416).

**Table 1:** The atomic fraction of the UV-grafted PDMS layers on anodized stainless steel measured by energy-dispersive X-ray spectroscopy.

| Time / Element | 0 min | 5 min | 15 min | 30 min | 60 min |
|---|---|---|---|---|---|
| **C** | 0.92 | 6.09 | 5.35 | 6.63 | 6.84 |
| **O** | 34.15 | 25.95 | 36.66 | 30.24 | 33.71 |
| **Si** | 0 | 3.23 | 4.85 | 3.55 | 5.09 |
| **Mo** | 0.86 | 0.73 | 0.61 | 0.72 | 0.62 |
| **Cr** | 13.59 | 12.43 | 12.42 | 11.71 | 11.55 |
| **Fe** | 36.9 | 37.39 | 29.59 | 35.18 | 31.46 |
| **Ni** | 4.76 | 4.66 | 4.03 | 4.51 | 4.07 |
| **F*** | 8.81 | 9.52 | 6.49 | 7.47 | 6.66 |

* F ions present in the anodization electrolyte.

The PDMS grafting procedure can be applied virtually to any material. Here, we examined the following substrates: (i) plain Si, and (ii) Si wafer covered by 100-nm-thick $SiO_2$ layer, (iii) 1-mm-thick standard microscope glass slides, 0.4-mm-thick borosilicate glass coverslips, and fluorine-doped tin oxide (FTO) conductive glass, and quartz, (iv) bare Co, and (v) Mo foils, (vi) polished Al, and Cu, (vii) vertically-aligned highly-ordered anodized $TiO_2$ nanotubular arrays ($TiO_2$ NTs((A. B. Tesler, M. Altomare, P. Schmuki, ACS Appl. Nano Mater. 2020, 3, 10646) and (viii) anodized 316L grade stainless steel with sponge-like nanoporous structure. The WCA measured on flat and rough substrates was hydrophilic for all plain samples (Figure 1e-f). The grafted samples show the water contact angles as a function of the surface roughness (Figure 1g). All flat grafted samples demonstrate a WCA in the range of 110 $\pm$ 4°, while rough substrates display 115° $\pm$ 3°, 140° $\pm$ 1°, 147° $\pm$ 3°, and 148° $\pm$ 3° for FTO, $TiO_2$ NTs, pseudoboehmite aluminum oxide, and anodized stainless steel, respectively.

The influence of PDMS oil with increased viscosity on the wetting characteristics of the UV-grafted surfaces was studied on flat Si substrates. The overall thickness of the grafted PDMS layer increased with a rise in PDMS oil viscosity from 5.5 $\pm$ 0.2 nm for 100 cSt ($M_w$ = 6.0 kDa) oil to 7.4 $\pm$ 0.1 nm and 11.3 $\pm$ 0.9 nm for 500 cSt ($M_w$ = 17.3 kDa) and 1000 cSt ($M_w$ = 28 kDa), respectively. However, only a minor increase in WCA with an increase in oil viscosity was observed (Figure 1h). However, the obtained UV-grafted PDMS layer thicknesses differ substantially from the water-assisted and photo-catalytically grafted procedures. For instance, Liu *et al.* demonstrated that UV-grafted PDMS on photocatalytic $TiO_2$ layer, the thicknesses of 2.2, 4.0, and 5.5 nm were obtained for 6.0, 17.3, and 28 kDa PDMS oils, respectively (J. Liu, L. Ye, S. Wooh, M. Kappl, W. Steffen, H.-J. Butt, ACS Appl. Mater. Interfaces 2019, 11, 27422). The grafted PDMS layer thickness was then displayed vs. molecular weight using a *log-log* plot. A straight line with a slope of $H$ = 0.43 $\pm$ 0.14 was obtained (Figure 2). The grafting density can be calculated by $\sigma = (H\rho N_A)/M_w$, where $\rho$ is the bulk density of polymer and $N_A$ is Avogadro's number (J. Liu et al, loc. cit.) Grafting densities of 5.3, 2.5, and 2.3 $\times 10^{17}$ molecules per $m^2$ were obtained for 6.0, 17.3, and 28 kDa PDMS, respectively. These results correlate with the literature indicating that the higher $M_w$ PDMS molecules occupy a larger area, thus increase the barrier for the next arriving molecule and, by that, reducing the grafting density. Nevertheless, the grafting density obtained in this study is doubled compared to thermal PDMS-grafting (L. Chen, S. Park, J. Yoo, H. Hwang, H. Kim, J. Lee, J. Hong, S. Wooh, Adv. Mater. Interfaces 2020, 7, 2000305), and to that obtained on photocatalytic $TiO_2$ substrates (S. Wooh, N. Encinas, D. Vollmer, H.-J. Butt, Adv. Mater. 2017, 29, 1604637). Without wishing to be bound by theory, the latter can be attributed to the non-specific dissociation of PDMS molecules occurring on the photocatalytic substrates, while in absence of photoactive material, this probability diminishes.

Several studies evaluated the photo-dissociation of silicon elastomers (a) B. Schnyder, T. Lippert, R. Kötz, A. Wokaun, V.-M. Graubner, O. Nuyken, Surf. Sci. 2003, 532-535, 1067; b) M. Brinkmann, V. Z. H. Chan, E. L. Thomas, V. Y. Lee, R. D. Miller, N. Hadjichristidis, A. Avgeropoulos, Chem. Mater. 2001, 13, 967; c) M. Ouyang, R. J. Muisener, A. Boulares, J. T. Koberstein, J. Membr. Sci. 2000, 177, 177). In these cases, however, UV light with $\lambda$ < 254 nm was utilized to prepare a permeable $SiO_2$ layer on cross-linked polysiloxanes, the present invention uses the induction of selective grafting processes. To address this issue, density functional theory (DFT) calculations on a PDMS truncated model molecule $Me_3Si$-O-$SiMe_2$-O-$SiMe_3$ were carried out, the results of which are shown in Table 2 below. Bond-dissociation enthalpies for breaking the molecule at different positions into two fragments were calculated as a measure of probability for a dissociation process. According to calculations, the dissociation enthalpy for Si-Me bonds is the smallest, and thus these bonds are generally weaker and more likely to break. UV light $\lambda$ < 321 nm (UVA) is required for photo-dissociation. The Si-O bonds are the strongest, and, therefore, harder and less expected to break, while stronger UV light $\lambda$ < 214 nm (UVC) is required in this case (Table 2). Consequently, in the case of selective grafting

using mild UV light, silicone oil is expected to be activated at Si-Me bonds being grafted to surfaces *via* silicon. A spectrum of a suitable medium pressure UV lamp as used in this experimental part is shown in Figure 3. As shown, the highest intensity peaks are observed at $\lambda$ = 320 and 370 nm indicating that the lamp provides a sufficient amount of energy at $\lambda$ = 321 nm to dissociate Si-Me bonds.

**Table 2:** Calculated bond dissociation energies and corresponding wavelengths required for dissociation of a $Si_3$-truncated model of polydimethylsiloxane (silicone oil) at given locations. Theory level: DFT/M062X/def2-TZVP, zero-point, and thermal energy corrections have been applied.

| Molecular structures with bonds to be dissociated | Bond dissociation enthalpy $DH^0_{298}$, [kcal mol$^{-1}$] | Appropriate wavelength for dissociation, [nm] |
|---|---|---|
| 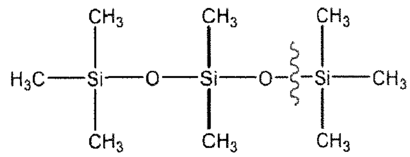 | 133.5 | 214 |
| 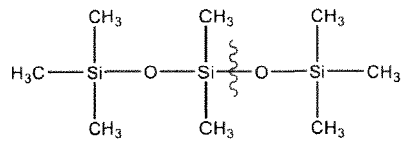 | 138.5 | 207 |
| 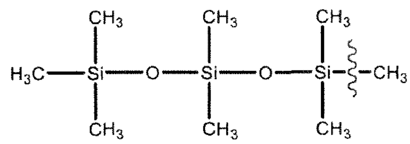 | 89.0 | 321 |
| 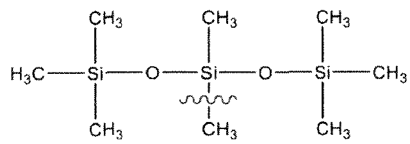 | 90.4 | 317 |
| 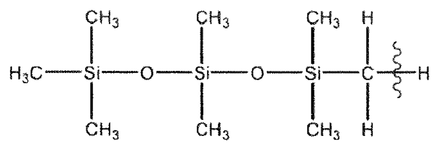 | 96.5 | 296 |

To study the chemical composition as well as surface coverage of UV-grafted PDMS layers, angle-resolved X-ray photoelectron (XPS) spectroscopy analysis was performed at angles of 15°, 45° and 75°, namely to control the detection depth in a sample by changing the sample's tilt angle. PDMS oils of 100, 500, and 1000 cSt viscosity have been grafted on polished 316L grade stainless steel substrates, and the representative high-resolution XPS spectra are shown in **Figure 4a-d.** An austenitic stainless steel alloy was measured as a reference showing the typical Fe, Cr, Ni, Mo, O, and C peaks (S. Tardio, M.-L. Abel, R. H. Carr, J. E. Castle, J. F. Watts, J. Vac. Sci. Technol., A 2015, 33, 05E122). When UV-grafted, the survey XPS spectra consist of Si, O, and C peaks associated with PDMS, while the Fe 2p peak arising from the substrate is barely noticeable. Still, the Fe 2p peak is clearly shown in the high-resolution XPS spectrum measured at 75° angle, and it completely disappears at 15° angle confirming that the PDMS grafted layer covers the substrate entirely (Figure 4d). The high-resolution Si 2p XPS spectrum consists of a single peak at the binding energy of 102.2 eV (Figure 4a), which can be deconvoluted into two components: at 102.2 eV (35 %) and 102.8 eV (65 %) corresponding to Si-C and Si-O bonds, respectively (A. Kaur, P. Chahal, T. Hogan, IEEE Electron Device Lett. 2016, 37, 142). In the case of the O 1s spectra, the main peak centered at 532.6 eV, and a shoulder at 530.5 eV is clearly visible. This shoulder is highest at 75° angle (Figure 4b) and disappears at angle of 15° (Figure 4b). Further deconvolution of the O 1s peak at 75° angle of the 100 cSt UV-grafted PDMS, leads to three components: at 532.6 eV (83.5 %) corresponding to the Si-O-Si bond (A. J. Pertsin, M. M. Gorelova, V. Y. Levin, L. I. Makarova, J. Appl. Polym. Sci. 1992, 45, 1195) and 531.1 eV (2.1 %), and 530.3 eV (14.4 %) corresponding to Me-O-Si and Me-O-Me bonds, respectively, where Me represents Fe, Ni, or Cr, *i.e.* main components of the substrate (S. Wooh, N. Encinas, D. Vollmer, H.-J. Butt, Adv. Mater. 2017, 29, 1604637). When measured at a 15° angle, only two components are observed: 532.6 eV (98.1

%) and 530.3 eV (1.9 %) assigned for Si-O-Si and Me-O-Me bonds, respectively, i.e. the peak attributed to Me-O-Si disappears. The latter results indicate again a complete coverage of the surface by the grafted PDMS molecules. A similar tendency was also observed for the higher molecular weight PDMS UV-grafted to stainless steel substrates, whilst the Me-O-Me peak was barely noticeable even at 75° angle. The C 1s spectra display a single peak at 285.0 eV, which is associated with Si-C bonds in PDMS oil (Figure 4c) (A. Sabata, W. J. van Ooij, H. K. Yasuda, Surf. Interface Anal. 1993, 20, 845). Furthermore, the Si and Fe XPS sputter profiles of UV-grafted PDMS oil of various viscosities demonstrate that the thickness of the grafted PDMS layers increases with oil viscosity (Figure 4e), correlating well with ellipsometric measurements (Figure 1h).

Time of flight secondary ion mass spectrometry (ToF-SIMS) analysis was performed for the UV-grafted PDMS layers deposited on AISI 316L grade stainless steel substrates, and the results are summarized in Figure 4f. The positive and negative spectra confirm the coverage of the surface by PDMS layer, as typical mass peaks are observed for PDMS (A. Sabata, W. J. van Ooij, H. K. Yasuda, Surf. Interface Anal. 1993, 20, 845), *e.g.* with positive polarity +43, +73, +147, +207 amu. A difference in the $Si^+$, $O^-$, $Si^-$ mass fragment intensities is also observed for the UV-grafted PDMS layers with different viscosities.

To further investigate the UV-grafted PDMS layer, negative depth profile obtained by ToF-SIMS analysis of the UV-grafted 100 cSt PDMS oil for selected representative fragments ($C^-$, $O^-$, $Si^-$, $Ni^-$, and $Fe^-$ signals) is presented in Figure 4e. The depth profile is listed as signal intensity for the sputtering time, since converting the sputtering time of ToF-SIMS into depth is difficult as the sputtering rate depends on the material; thus the sputtering time serves as a qualitative measure of depth (Y. Hashimoto, T. Yamamoto, Appl. Surf. Sci. 2017, 419, 319). The interface between the PDMS layer and the substrate is reached at approximately 140 s, as after this time the increasing intensity of the $Fe^-$ and $Ni^-$ signals reaches a plateau, i.e. sputtering of the substrate sample. The $Si^-$ signal is representative of the PDMS layer, and the intensity of $Si^-$ initially increases during the first 40 s, then decreases continuously till 140 s of sputtering, and a steady-state value is reached (Si is present in the austenitic grade steels as impurity). The intensity of $O^-$ and $C^-$ is more complex as the concentration of $O^-$ initially increases for 60 s of sputtering, and then decreases continuously (due to the native oxide layer), while for $C^-$ it decreases for 40 s and then starts increasing (a bump at 120 s of sputtering) and, finally, reaches a steady condition at 300 s. The latter may be explained by the presence of carbon in the austenitic grade stainless steel substrate. The initial low intensities of $Fe^-$ and $Ni^-$ corroborate the high coverage of the solid substrate by the UV-grafted PDMS layer. In particular, the variance in the intensities of $C^-$, $O^-$, and $Si^-$ signals can point to the preferential orientation of the low viscosity UV-grafted PDMS molecules on the solid substrate, while the top trimethylsiloxane group is initially removed exposing O and Si atoms.

**2.2 Corrosion resistance of UV-grafted PDMS on metallic substrates.**

Corrosion is a natural process of metal deterioration, driven by its reaction to the surrounding environment, and is the main reason for the failure of metal structures. The need to minimize the damage caused by corrosion is particularly evident when it comes to engineering materials such as Al, Cu, and various grades of steel.

As an example, the corrosion resistance of plain, UV-grafted, and PDMS-infused flat Al substrates was evaluated by dropping 1 M NaOH aqueous solution on their surfaces. The plain and UV-grafted samples react almost immediately with the corrosive solution, while the PDMS-infused sample displays an approximately 10 min delay in the corrosion initiation being comparable with the thermally-grafted PDMS layers (L. Chen, S. Park, J. Yoo, H. Hwang, H. Kim, J. Lee, J. Hong, S. Wooh, Adv. Mater. Interfaces 2020, 7, 2000305. After that time, $H_2$ bubbles corresponding to $H^+$ cathodic reduction appear also on the PDMS-grafted sample, yet the etching solution preserves its drop shape restricting the fast spreading of the corrosive media over the entire sample surface (Figure 5a). After 15 min, the corroded area fraction was calculated and found to be 96%, 43%, and 18% for the plain, UV-grafted, and PDMS-infused Al, respectively (Figure 5b). Such a facile dropping test demonstrates not only a delay in corrosion initiation of the PDMS-infused samples but also limits the spreading of the corrosive medium on the PDMS-infused surface indicating robust corrosion resistance.

As a next step, standard potentiodynamic polarization measurements for Al, Cu, and AISI 316L grade stainless steel were performed in a standard 3.5 wt.% NaCl electrolyte solution. The corrosion susceptibility decreases when stainless steel, Al, and Cu substrates are UV-grafted with PDMS oil. Stainless steel and Al show a passive behavior until the onset of pitting corrosion, which is increased towards higher potentials after the surface grafting. Cu shows active dissolution starting with an activation-controlled region followed by a diffusion-controlled plateau with higher current densities. The corrosion potential of the three materials is increased, while the corrosion current is at least one order of magnitude lower in the case of stainless steel and Cu. On the other hand, Al shows the presence of metastable pitting until the onset of pitting corrosion is reached.

The effect of UV-grafted PDMS layer on the corrosion of metal substrates was quantified by the potentiodynamic polarization measurements. The corrosion current densities ($I_{corr}$) were estimated using Tafel plots, and the results are summarized in Table 3 below. The corrosion current density of the UV-grafted steel is one order of magnitude lower than that of bare steel. This indicates that the grafting of PDMS coating suppresses the corrosion of stainless steel with

a corrosion inhibition efficiency of 96.8 %. Lubricant-infused slippery samples fabricated by grafting a PDMS layer and without removing the remnant oil showed an even higher corrosion resistance. The noisy signal near the open circuit potential (OCP) in both, cathodic and anodic range, is given by the low current density, with values near the equipment scale limit. The passive current density values are similar to the UV-grafted substrate. Also, when a potential near the pitting potential of the bare substrate is reached, current spikes start to appear until the onset of pitting corrosion at *circa* 1.0 V. The fact that these spikes reach such high current values indicate that localized events similar to metastable pitting corrosion could take place, but in this case, instead of re-passivation, a self-healing effect of the liquid coating occurs covering these sites, hence lowering the current until a new location is attacked.

Potentiodynamic polarization of bare anodized stainless steel in the chloride-containing electrolyte shows a higher $I_{corr}$, a lower $E_{corr}$, and a shorter passivation range due to the onset of pitting corrosion when compared to the bare stainless steel substrate (Table 3). Pitting corrosion was also evident by the localized dissolution of the anodized oxide layer, while the underlying metallic substrate could be observed. Corrosion behavior of anodized stainless steel substrates has been previously reported (L. Prado, E. Anastasiou, S. Virtanen, J. Electrochem. Soc. 2021, 168, 021507.). However, the anodized and UV-grafted substrate presented a strong decrease in both $I_{corr}$ and $E_{corr}$. When the anodized stainless steel samples were further infused by PDMS lubricant, an additional order of magnitude decrease of the corrosion current density is observed in comparison to the anodized UV-grafted steel. Thus, both PDMS coated samples demonstrate almost 100% corrosion inhibition efficiency compared to the bare anodized stainless steel substrate. Furthermore, the passivation range was once again increased, proving that the grafted PDMS layer and the infused coating highly decrease the susceptibility to pitting corrosion. A similar trend was also observed in medical-grade instruments made of martensitic stainless and carbon steel, which is prone to corrode in saline solutions. As we show below, the corrosion resistance of the UV-grafted carbon grade steel blades improves substantially, while its plain analog corrodes almost immediately, when both were exposed to the corrosive bacteria culture medium consisting of 0.1 wt.% NaCl.

Finally, we examined corrosion resistance of Al and Cu, widely used in industrial applications metals, which are prone to corrode when exposed to corrosive chemicals. The Cu substrate UV-grafted with PDMS oil demonstrated a corrosion inhibition efficiency of 94.8 %, while the corrosion current of the Al UV-grafted sample was at the same level as the bare Al substrate. When infused with silicone oil, the same samples show 98.1 % and 99.9 % corrosion inhibition efficiency for Al and Cu, respectively. Here, the addition of silicone lubricant forms a homogeneous thin layer on the UV-grafted PDMS layer due to the matching surface energies that serves as a passivation layer inhibiting direct contact between the corrosive media and the solid substrate. As shown, the coating process can be efficiently applied on various plain metal surfaces without porous structuring or other pre-treatments. However, as demonstrated by potentiodynamic diagrams, the porous PDMS-infused surfaces demonstrate an additional enhancement in the corrosion resistance, while micro/nano-structuring of the substrate certainly compromises its mechanical characteristics. Therefore, the compromise between surface structuring of the metal surface, *i.e.* mechanical properties, and its corrosion resistance characteristics should be considered depending on the metal application requests.

**Table 3:** The electrochemical parameters of polished bare 316L grade stainless steel, modified by 30 min illumination polished PDMS steel (hydrophobic), anodized steel with PDMS coating (superhydrophobic), and lubricated anodized steel with PDMS coating measured by the potentiodynamic polarization curve.

| System | $E_{Corr}$ (mV) | $I_{Corr}$ (A $cm^{-2}$) | $\beta$a (mV) | -$\beta$c (mV) |
|---|---|---|---|---|
| Polished bare StSt | -193 | $6.60\times10^{-8}$ | 140 | 79 |
| Polished StSt/UV-grafted | 57 | $2.08\times10^{-9}$ | 141 | 70 |
| Polished StSt/PDMS-infused* | 144 | $2.15\times10^{-10}$ | 205 | 209 |
| Anodized bare StSt | -22 | $2.93\times10^{-6}$ | 217 | 94 |
| Anodized StSt/UV-grafted (Superhydrophobic) | 310 | $2.15\times10^{-9}$ | 75 | 56 |
| Anodized StSt/PDMS-infused | 396 | $3.90\times10^{-10}$ | 93 | 76 |
| Polished Al | -692 | $1.24\times10^{-7}$ | 168 | 164 |
| Polished Al/UV-grafted | -634 | $5.57\times10^{-7}$ | 85 | 51 |
| Polished Al/PDMS-infused | -911 | $2.38\times10^{-9}$ | 260 | 397 |
| Polished Cu | -296 | $1.12\times10^{-5}$ | 73 | 79 |
| Polished Cu/UV-grafted | -164 | $5.87\times10^{-7}$ | 48 | 46 |

(continued)

| System | $E_{Corr}$ (mV) | $I_{Corr}$ (A cm$^{-2}$) | $\beta$a (mV) | -$\beta$c (mV) |
|---|---|---|---|---|
| Polished Cu/PDMS-infused | -316 | $2.64\times10^{-9}$ | 79 | 121 |
| *The values are approximated due to the high level of noise. | | | | |

**2.3 PDMS UV-grafted process applied on medically applicable surfaces.**

*2.3.1 Blood-repellent properties of UV-grafted PDMS layers on surgical devices.*

Surgical blades can be manufactured from either carbon- or martensitic stainless steel, both of which have to comply with the requirements of BS 2982:1992 or BS EN ISO 7153 Part 1 standards that specify hardness and material composition (a) BS-EN-ISO-7153, in *Part 1: Metals,* The International Organization for Standardization, 2016; b) BS-2982:1992, The British Standards Institution, 1992, 10). This is to make sure that the blades are fairly corrosion-resistant, have a high degree of strength, shock-resistant, and in some particular cases are non-magnetic. Here, carbon steel blades offer a better combination of initial sharpness and durability, while at the same time they rust almost immediately when coming into contact with saline solutions (D. Dwivedi, K. Lepková, T. Becker, RSC Adv. 2017, 7, 4580) (Figure 6). Therefore, the influence of UV-grafted PDMS coating in both "as-grafted" and lubricant-infused configurations on the corrosion resistance blade grades was examined using the potentiodynamic polarization technique.

Potentiodynamic polarization of plain carbon- (CS) and martensitic stainless steel (MSS) scalpel blades, UV-grafted with PDMS oil and PDMS-infused show a clear decrease of corrosion susceptibility (Figure 7a-b and Table 4). The carbon steel blade presents a higher corrosion potential and a decrease in corrosion current with a 69.5 % corrosion inhibition efficiency in the case of CS blades grafted and further infused with PDMS oil. Furthermore, the onset of active dissolution is delayed toward higher potentials. The corrosion resistance of MSS blades is improved even further by the PDMS coatings. This could indicate a higher quality of the coating due to the hydroxide reach layer on the surface of the native passive oxide, which has more hydroxide bonds available to react with the PDMS molecules. MSS blades grafted with PDMS oil showed a 98.8% corrosion inhibition efficiency, whilst with MSS blades (grafted and infused) not only the corrosion potential of the blade is lower, but also the current density in the passive region, hence generating a noisy signal of $10^{-9}$ A cm$^{-2}$. Additionally, a significant delay in pitting corrosion onset is observed.

**Table 4:** The electrochemical parameters of plain carbon (CS) and martensitic stainless steel (MSS) scalpel blades, UV-grafted by 30 min illumination (hydrophobic), and PDMS-infused coating measured by the potentiodynamic polarization curve.

| System | $E_{Corr}$ (mV) | $I_{Corr}$ (A cm$^{-2}$) | $\beta$a (mV) | -$\beta$c (mV) |
|---|---|---|---|---|
| Bare CS Blade | -723 | $6.3\times10^{-7}$ | 50 | 160 |
| CS Blade/UV-grafted | -646 | $1.34\times10^{-6}$ | 112 | 188 |
| CS Blade/PDMS-infused | -497 | $1.92\times10^{-7}$ | 73 | 151 |
| Bare MSS Blade | -284 | $4.56\times10^{-7}$ | 71 | 109 |
| MSS Blade/UV-grafted | -99 | $5.37\times10^{-9}$ | 20 | 75 |
| MSS Blade/PDMS-infused* | - | - | - | - |
| * Due to the very small current signal, it was impossible to calculate the electrochemical parameters. | | | | |

In invasive medical procedures, the scalpel blade surface experiences significant friction made through the skin and subcutaneous tissue incision. At the same time, these instruments are exposed to pathogens on the patient's skin and blood, potentially leading to infection. Therefore, the performance of the UV-grafted PDMS coating was evaluated first by making an incision with the treated blades into viscoelastic silicone rubber slabs of various softness and stickiness (polydimethylsiloxane (PDMS) base/curing agent ratios of 30:1, 20:1, and 10:1, SYLGARD® 184) chosen to mimic skin tissues (A. B. Tesler, P. Kim, S. Kolle, C. Howell, O. Ahanotu, J. Aizenberg, Nat. Commun. 2015, 6, 8649). This was followed by submersion into whole blood and medium containing *E. coli and* S. *aureus.*

In controls, i.e. untreated scalpel, the softest slab (30:1) stuck to the scalpel surface and moves along the direction of the cut creating an uneven incision in both scalpel types (Figure 7c, top row). For the hardest slab (10:1), it

was very difficult to cut through it because of the high friction. In all cases, incisions produced by untreated scalpels tend to have non-uniform cuts and fractures due to the stick-slip-like, jerking motion as observed by the appearance of perpendicular lines periodical to the scalpel motion. In contrast, the UV-grafted and PDMS-infused blades produced nearly identical incisions on all slabs (Figure 7c-d, middle, and bottom rows), which are as smooth as the scalpel blade edge roughness.

When medical devices come into contact with blood, the interaction poses a threat of undesired activation of blood cells (platelets and monocyte/macrophages of the immune system) or other bioactive blood components (complements of coagulation cascades) potentially leading to the formation of blood clots, or thrombi, inflammation, or a more widespread, prolonged activation of the immune system. Therefore, after the incision experiments, treated and control scalpels of both grades were submerged in a cuvette with fresh blood (Figure 7e-f). After 10 s of submersion, the scalpels were withdrawn from the cuvette with blood and washed in a cuvette filled with 4 mL of phosphate buffer saline (PBS). The amount of adherent blood to UV-grafted and PDMS-infused scalpels was substantially lower as indicated by the color of the cuvette filled with PBS and by UV-Vis spectroscopy (Figure 7e-f). The bare scalpels demonstrate a significant amount of attached blood in both cases as shown by the remaining blood on its surface and the pinned buffer film after the wash in PBS. The PDMS-infused scalpels display substantial reduction or even the absence of the peaks associated with hemoglobin (Figure 7e-f, bottom spectra) (E. K. Hanson, J. Ballantyne, PLoS One 2010, 5, e12830). The superior blood repellent performance is attributed again to the existence of a stable liquid-liquid interface, formed between the low-surface-tension liquid and the blood, which is contrasted by the easily contaminated solid surface present of bare scalpels. Due to the present invention, this important anti-fouling characteristic can now also be applied on a bare non-porous substrate without compromising mechanical characteristics of bulk material. Even UV-grafted PDMS scalpel of both steel grades sustained their repellent characteristics after submersion in blood, demonstrating a decrease in adhesive blood (Figure 7e-f, middle and topmost spectra).

Over the years, the field of optical instrumentation in biomedical applications has evolved due to the constantly growing demand for minimally invasive surgical procedures such as laparoscopy and diagnostic measurements such as endoscopy. While laparoscopic cholecystectomy rapidly became the standard of care for cholelithiasis after 1988, the use of minimally invasive techniques for other abdominal operations is yet limited (G. H. Ballantyne, Surg. Laparosc. Endosc. Percutan. Tech. 2002, 12, 1). Several inherent pitfalls of laparoscopy hinder the performance of these operations, including the limited motion of straight laparoscopic instruments, the narrow field of view, and poor visibility due to bleeding (T. Okamoto, T. Ohnishi, H. Kawahira, O. Dergachyava, P. Jannin, H. Haneishi, Signal Image Video P. 2019, 13, 405). Surgical laparoscopes used for minimally invasive procedures consist of hollow tubes made of high-quality stainless steel that gradually developed to include fixed glass lenses for magnified vision. Modern scopes are designed with multiple parts including a CCD camera, viewing, and energy-supply devices, and a lens cleaner to mechanically wipe off blood to improve the field of view. Several attempts have been made to create glass surfaces slippery, while in all these cases the surface was first roughened by micro/nano structures (N. Vogel, R. A. Belisle, B. Hatton, T.-S. Wong, J. Aizenberg, Nat. Commun. 2013, 4, 2176; S. Sunny, G. Cheng, D. Daniel, P. Lo, S. Ochoa, C. Howell, N. Vogel, A. Majid, J. Aizenberg, Proc. Natl. Acad. Sci. U. S. A. 2016, 113, 11676). The latter, however, alternates dramatically the mechanical characteristics of the prepared slippery liquid-infused glass substrates. Once damaged due to the mechanical abrasion, which frequently occurs during the surgery, it would lose slipperiness further altering its blood-repellent characteristics (N. Vogel et al., loc. cit.). Therefore, considering that, the structuring process is rather complicated and the mechanical robustness is weak, both these factors limit the future application of slippery liquid-infused porous glass surfaces in real devices.

As demonstrated above, various grades of steel can be UV-grafted by silicone oil, which prevents blood adhesion as well as making incisions smoother. The wetting characteristics were preserved on both structured and flat substrates without deteriorating the mechanical characteristics. We applied therefore our UV-grafting procedure on the second functional component of the laparoscope that interacts with human tissue, *i.e.* magnifying glass lenses to improve the field of view in a passive form, *i.e.* without application of any external stimuli or energy (Figure 7g-k). Figure 7g shows bare (left lens), PDMS UV-grafted (middle lens), and PDMS-infused (right lens) lenses of comparable transparency. However, the wetting characteristics of bare, and UV-grafted or PDMS-infused lenses differ substantially. Fresh blood (500 $\mu$L) was added dropwise on every lens. An increased amount of blood finally led to the percolation of individual droplets covering -27 % of the bare hydrophilic lens surface (Figure 7h-i, left lens). At the same time, UV-grafted and PDMS-infused samples, which are hydrophobic, prevent percolation of blood droplets, which cover only ~14 % of the lens surface in both cases (Figure 7h, middle and right lenses). Nevertheless, there is a substantial difference also between the UV-grafted and PDMS-infused lenses. On the UV-grafted lens, blood remains static independently of the lens curvature (Figure 7i, middle lens), while it immediately slides off on the PDMS-infused lens. The sliding velocity depends on the curvature, as it takes about 40 s for the droplets to slide off completely from the top of the lens leaving behind a completely clear field of view (Figure 7j). Afterward, the lenses were tilted vertically to remove the blood, and the covered area fraction was calculated and found 35 %, 3.58 %, and 0.46 % for the bare, UV-grafted, and PDMS-infused lenses, respectively (Figure 7k). The latter indicates that such a facile treatment of laparoscope functional

surfaces can significantly improve its field of view without the image distortion as well as keeping its mechanical characteristics. Furthermore, the device can be mechanically wiped and chemically cleaned after the medical treatments, then re-grafted using the same procedure by simultaneously sterilizing it by intense UV-light illumination.

*2.3.2 Anti-bacterial properties of UV-grafted PDMS layers on surgical devices.*

The second issue that should be considered in biomedical applications is the existence of various types of bacteria on the human skin. It is well-known that surgical site infections (SSIs) are among the most common healthcare-associated infections, which are associated with longer postoperative hospital stays, additional surgical procedures, treatment in intensive care, and higher mortality. The percentage of SSIs can vary from 0.5 % to 10 %, depending on the type of surgical procedure, while a statistically significant increasing trend was observed for both the percentage of SSIs and the incidence density of in-hospital SSIs following laparoscopic cholecystectomy in recent years. Thus, the process of bacterial adhesion on steel substrates was also examined. To demonstrate the antibacterial characteristics of the UV-grafted surfaces, *E. coli* (Gram-negative) and *S. aureus* (Gram-positive) bacteria were cultured on bare, polished, UV-grafted, and PDMS-infused substrates as well as on anodized porous PDMS-infused steel substrates. These two types of bacteria are frequently found on human skin. All the substrates had a flat morphology without pores or textured structures except the anodized ones. Initially, the samples were sterilized in a 70 vol.% ethanol solution and then incubated in bacterial solutions to analyze bacterial attachment. After 24 h, the samples were withdrawn from the growing bacterial solution, gently washed with DI water then stained by crystal violet dye (Y. Kovalenko, I. Sotiri, J. V. I. Timonen, J. C. Overton, G. Holmes, J. Aizenberg, C. Howell, Adv. Healthc. Mater. 2017, 6, 1600948. The results are presented in Figure 8a-l. The surface coverage was calculated using bright-field microscopy in reflectance mode as shown in Figure 8a-j and backed up by confocal fluorescence microscopy and SEM imaging (Figure 9). As shown, both bacteria biofilms are formed on bare and polished steel substrates resulting in purple color over their entire surface due to crystal violet staining. The surface coverage of the bare hydrophilic steel substrates was estimated as 80 $\pm$ 5 % and 78 $\pm$ 8 % for *E. coli* and S. *aureus* biofilms, respectively; on the polished steel samples it was reduced to 64 $\pm$ 16 % for *E. coli* ($p$ = 0.082) and 49 $\pm$ 17 % for *S. aureus* ($p$ = 0.030) biofilm coverage (Figure 8a-b, f-g). According to the SEM images, the formed biofilms consist mainly of a single layer of bacteria (Figure 9a, c). The UV-grafted PDMS samples already revealed a significant reduction in the biofilm surface coverage displaying 22 $\pm$ 13 % and 17 $\pm$ 5 % for E. *coli* and *S. aureus,* respectively ($p$ < 0.005). There are, however, substantial differences in the biofilms formed on the UV-grafted substrates. According to the SEM images, biofilms formed on the UV-grafted samples consist of multilayer structures (Figure 9b, d). This difference is attributed to considerably weaker adhesion of both types of bacteria to the grafted surface, while the drug applied by the capillary force due to the evaporation of water is enough to detach bacteria from the substrate and condense them into multilayered clusters. At the same time, the PDMS-infused samples exhibit outstanding resistance against bacteria inhibiting almost complete adhesion of biofilms to the substrate surface ($p$ << 0.005) (Figure 8d-e, i-j). The attachment and biofilm formation for the *E. coli* and S. *aureus* are suppressed by >97 % on flat and >99 % on the anodized porous PDMS-infused steel substrates. The immobilized low-surface-tension PDMS lubricant stably present on the UV-grafted and infused substrates, as shown by the thin-film interference pattern in Figure 8d, e, j, introduces an additional liquid-liquid interface, which effectively eliminates the adhesion of both types of bacteria forming biofilm resistive coating.

Next, the UV-grafting approach was applied on the bare, UV-grafted, and PDMS-infused scalpel blades to examine the adhesion of both *E. coli* and *S. aureus* in a medium. After sterilization by dipping the blades in 70 vol.% ethanol solution for a few seconds and drying them at ambient atmosphere, the blades were dipped in a solution of medium containing approx. 8 $\times$ $10^8$ cells $mL^{-1}$ ($OD_{600nm}$ = ~1.000) bacteria. The blades were kept for 1 min in the bacterial solution, then withdrawn and rinsed in a cuvette with bacterial medium to remove unbound bacteria, *i.e.* from the droplets that pinned to the blade surface. The cuvette with rinsed bacteria was then measured by UV-Vis spectroscopy at $\lambda$ = 600 nm ($OD_{600nm}$), and the results are summarized in Figure 8m-n (J. A. Myers, B. S. Curtis, W. R. Curtis, BMC Biophysics 2013, 6, 4). The amount of remnant bacteria was reduced from bare to UV-grafted and PDMS-infused blades.

Immediately after the rinse in the bacterial medium, the incision of the agar plate was performed to determine the number of bacteria that remain on the blade even after extensive rinse, i.e. bacteria that strongly adhere to the blade surface. The agar plates were then incubated at 37 °C for 24 h allowing bacterial growth, and the results are presented in Figure 8m-n, top inset images. The number of adherent bacteria to the PDMS-infused scalpels is approximately 60 % lower for *S. aureus* and about 50 % for *E. coli* compared to plain blades. Furthermore, there is a substantial difference between plain and UV-grafted or PDMS-infused blades in both steel grades. The bacterial biofilm grown from all plain scalpels is even and continuous over the entire incision length (Figure 8m-n, left petri plates in the inset images). On the contrary, UV-grafted and, in particular, PDMS-infused blades display a patchy growth of bacterial biofilms with a thicker grown area at the beginning of the incision. After approximately 2 cm, the biofilms rupture, and only a few individual colonies are observed. The latter indicates that the adhesion of the bacteria is considerably weaker in both UV-grafted and PDMS-infused blades, since the applied friction between the soft agar gel and scalpel blade is sufficient enough to

remove the attached bacteria almost completely, which does not occur on plain blades.

2.3.3 Anti-biofouling coating against marine species

Stainless steel is a widely used material in marine applications. However, in the marine environment steel is prone to biofouling, which interferes substantially with its desired characteristics. There are several methods to remove biofouling: (i) physical method such as mechanical cleaning with high-pressure water jets are costly, time-consuming, and non-effective. (ii) Chemical methods such as Tributyltin (TBT), copper- or zinc-based compounds and chlorination are toxic, not organism-specific and, therefore, have been partially banned from 2008 by the International Maritime Organization. (iii) A biological method such as the use of chemicals received from marine organisms is not proven as effective, expensive and not easily applicable. In this invention, we propose to struggle biofouling by fouling release (FR) coating made of the UV-grafted and lubricant-infused coating as a non-toxic anti-biofouling coating alternative.

The initial assessment of treatment performance evaluation was carried out using freshwater green alga of type *Chlamidontas reinhardtii (C. reinhardtii).* All samples from bare steel to polished, UV-grafted and lubricant-infused have been horizontally aligned in the culture medium and biofilm was growing on top of it. After 8 days of incubation, the samples have been removed from the medium and dried at an ambient atmosphere. The results for the remaining organisms on the treated and untreated samples are shown in Figure 10. As shown, bare and polished steel samples were covered by *C. reinhardtii* almost completely (Figure 10a-b). UV-grafted samples indicate a reduction in the biofilm attachment, which was observed by the gradual sliding down of the biofilm during the lifting process due to gravitational force (Figure 10c). The PDMS lubricant-infused samples were extremely resistive to biofilm attachment without application additional treatments showing ~100 % spontaneous delamination of the biofilm as the sample reaches the water-air interface (Figure 10d). Similar results have been observed also with the second algae of type *Chlorella sorokiniana.* In both these cases, the viability test was performed by measuring UV-vis spectroscopy at 600 nm wavelength ($OD_{600nm}$) *vs.* bare and polished stainless steel samples. In both cases, the $OD_{600nm}$ values were almost twice higher than that of bare and polished samples. For instance, the $OD_{600nm}$ value for the PDMS UV-grafted and lubricant-infused samples were 0.726 and 0.971, respectively, while the bare and polished samples showed values of 0.322 and 0.308, respectively.

In summary, the present invention as described above in general terms and by illustrating examples introduces a convenient approach to endow a surface of virtually any solid substrate with a coating providing superior repellent characteristics. It allows a lubricant-infused surface to be provided in a one-pot procedure. Thus, it has been demonstrated that a silicon oil such as polydimethylsiloxane (PDMS), a very common silicone material, grafts to a substrate when irradiated by UV light, and is able to act simultaneously as a surface energy reducing agent and infusing lubricant. The coating was applied to various substrates including metals, metal oxides, and ceramics with surface morphologies from flat to rough. The proposed approach is simple to implement, rapid, non-toxic, environmentally friendly, easily scalable, and low-cost, yet able to form a stable lubricant-infused slippery coating. Furthermore, the application of the coating does not require pre- or harsh treatment conditions, making it suitable to apply in industrial, medical, and other real-life applications. A key advantage of the approach is that micro- or micro/nano-surface structuring is not required. The latter thus eliminates the main complexity of superhydrophobic surfaces, which is an intrinsic mechanical weakness associated with rough surfaces, yet increases the resistance of the material to corrosion and fouling. Indeed, the addition of surface roughness through anodization and subsequent grafting of the surface with PDMS leads to superhydrophobicity. The latter further increases the corrosion resistance, broadens the passivation range, while the infusion of the superhydrophobic surface with silicone oil for creating a lubricant-infused slippery surface, further reduces the corrosion current density, and increases the corrosion potential. However, the structuring processes may apply harsh conditions on the material and are not always applicable for various substrates reducing the overall mechanical durability of the material. Therefore, a compromise between mechanical and repellent characteristics should be considered for every particular application.

Due to the biocompatibility of silicone-based compounds, the UV-grafting process can be favorably used, e.g. on medically applicable substrates to result in enhanced corrosion resistance, reduced friction, as well as excellent blood and bacterial adhesion repellency. The latter emphasizes that the surface properties of bare medical devices can be substantially modified without deteriorating their mechanical robustness. Furthermore, the UV-grafted medical instruments were sterilized by a brief rinse in 70 vol.% ethanol solutions and then were used several times without showing any degradation in their repelling characteristics. The procedure was applied on surgical grades steel scalpels that allowed to cut both soft and hard crosslinked elastomers more accurately. Furthermore, carbon steel, which is used frequently in medical applications, is prone to corrode almost immediately in saline solutions. When UV-grafted, the same scalpel blade made of carbon steel demonstrated substantial improvement in corrosion resistance. When the same procedure was applied on plain convex glass lenses, the slippery PDMS-infused layer was able to repel blood stains almost completely by passive sliding, allowing a complete field of view without distortion and without compromising the mechanical characteristics of bare lens material. The same trend was also observed for bacterial and marine green algae adhesion, while both Gram-positive and Gram-negative bacteria as well as marine species attach loosely to the

UV-grafted and PDMS-infused samples and can be removed by application of weak shear or even by capillary forces. Given all aforementioned advances, a wide range of practical applications can be envisaged for the presented approach.

Brief description of the Figures:

**Figure 1:** (**a**) Schematic representation of the PDMS grafting procedure to form PDMS-infused slippery surfaces. (**b**) The water contact angle (WCA) of a bare silicon wafer (left image), and WCA and contact angle hysteresis (CAH) of PDMS grafted Si wafer (the glow around the water drop in the right image represents the CAH obtained by the overlap between advancing and receding WCA images at 50 % transparency). (**c**) The PDMS layer thickness and corresponding WCA (line) and CAH (bars) values as a function of time of UV light illumination grafted on Si wafer. Insets: images of the actual water drop shape on the UV-grafted Si water. (**d**) EDX analysis of UV-grafted PDMS on polished stainless steel substrates as a function of UV light illumination time. (**e**) The WCA of bare (top row), PDMS-grafted (bottom row), and corresponding SEM images (middle row) of metallic substrates (from left to right): polished Al and Cu, bare Mo and Co, and polished stainless steel. (**f**) The WCA and corresponding SEM images of PDMS UV-grafted oxide surfaces (from left to right): 100-nm-thick $SiO_2$ layer on Si wafer, quartz, pseudoboehmite alumina (AlOx), fluorine-doped tin oxide glass (FTO), highly-ordered $TiO_2$ nanotubes ($TiO_2$ NTs), and sponge-like anodized 316L grade stainless steel (StStOx). The scale bar in (e-f) for all HR-SEM images is 100 nm. (**g**) WCA was measured before and after UV grafting of PDMS on the samples shown in (e-f), and (**h**) WCA and layer thickness measured by ellipsometry of grafted PDMS layers on Si wafer with increased PDMS oil viscosity.

**Figure 2:** The thickness of the UV-grafted PDMS layer on bare Si wafer surface as a function of the molecular weight.

**Figure 3:** Typical UV-vis spectrum of medium pressure mercury UV lamp.

**Figure 4:** High-resolution angle-resolved X-ray photoelectron spectroscopy spectra of UV-grafted 100 cSt PDMS oil on polished stainless steel substrates: (**a**) Si 2p, (**b**) O 1s, (**c**) C 1s, and (**d**) Fe 2p. (**e**) XPS depth profile of Si and Fe UV-grafted PDMS oil of various viscosities on polished stainless steel substrates. (**f**) ToF-SIMS depth profile of secondary ions derived from 100 cSt PDMS oil UV-grafted on polished stainless steel. The y-axis is in logarithmic scale.

**Figure 5.** (**a**) Digital still images of bare, UV-grafted, and PDMS-infused Al samples with a 1M NaOH aqueous solution drop. The images were captured after 10 min of corrosion. (**b**) The samples were dried 15 min after corrosion; the corroded area fraction was calculated, while the bright spots on the sample surface indicate the corroded area.

**Figure 6:** Still digital and optical microscope images of (**a**) bare and (**b**) UV-grafted by PDMS oil carbon steel scalpel blades after immersion for 1 min in tryptic soy broth containing 0.1 wt.% NaCl. The samples were dried and kept at ambient atmosphere. The images were captured after 1 h of submersion. The bare scalpel displays significant pitting corrosion, while the UV-grafted scalpel demonstrates no sign of corrosion.

**Figure 7:** (**a-b**) Potentiodynamic polarization of medical scalpel blade steel in 3.5 wt.% NaCl electrolyte of bare, PDMS UV-grafted, and PDMS-infused made of (a) martensitic stainless steel (MSS) and (b) carbon steel (CS). (**c-d**) Optical microscope cross-sectional images of incisions produced on 1-cm-thick, sliced PDMS slabs with increased softness and stickiness (base/curing agent ratio, indicated) by untreated (top row), UV-grafted PDMS (middle row), and lubricant-infused slippery (bottom row) scalpel blades made of (c) carbon and (d) martensitic stainless steel. (**e-f**) Digital still images and UV-Vis absorbance spectra of blood that adheres to the scalpel surface are shown in (a-b) after washing in phosphate buffer saline (PBS). (**g-i**) Still digital images of bare, PDMS UV-grafted, and PDMS-infused double-convex glass lenses (**g**) before, (**h**) immediately after dropping, and after (**i**) 40 s of exposure to 500 $\mu$L fresh blood. (**j**) Digital images of the lenses are shown in (g-i) after tilting by 90° to remove blood from the lens surface. (**k**) Coverage area fraction of bloodstains on glass lenses calculated for each lens in as-dropped (h) and after tilting by 90° (j) states.

**Figure 8:** Bright-field reflectance images of (**a-e**) *E. coli* and (**f-j**) S. *aureus* adhering to austenitic stainless steel substrates with the following modifications: (a, g) bare "as-received", (b, h) bare polished, (c, i) polished UV-grafted, (d, j) polished lubricant-infused, and (e, k) polished-anodized, and lubricant-infused. (**k-l**) Surface coverage area fraction calculated from the optical microscope images for all samples. Triplets were used for statistical analysis and at least two independent images were imaged of every sample. (**m-n**) Colony-forming unit counts for bare, UV-grafted, and PDMS-infused scalpel blades after dipping in a tryptic soy broth medium containing *E. coli* or *S. aureus* followed by extensive rinsing. Insets in (m, n) are representative images of the agar Petri dishes with incisions made by the bare, UV-grafted, and lubricant-infused scalpel blades. Scalpel blades (1-3) were used for every treatment, while the incision (4) was made by sterilized in 70 vol.% ethanol solution scalpel blades.

**Figure 9:** SEM images of (**a-b**) *Staphylococcus aureus* and (**c-d**) *Escherichia coli* after 24 h growth on 316L grade stainless steel samples. The bacteria were stained with crystal violet (CV) solution for 10 min, then gently rinsed and dried naturally.

**Figure 10:** Digital images of a freshwater green algae of type Chlamidontas reinhardtii (C. reinhardtii) after 8 days of incubation on (a) bare, (b) polished, (c) PDMS UV-grafted, and (d) PDMS lubricant-infused stanless steel samples. The samples have been removed from the medium, and dried at an ambient atmosphere.

## Claims

**1.** A process for the preparation of a silicone or fluorosilicone-coated solid substrate, which process comprises the following steps

a) providing a solid substrate having at least one surface to be coated,
b) bringing the surface to be coated in contact with a coating fluid comprising linear silicone or linear fluorosilicone molecules, and
c) irradiating the coating fluid in contact with the surface by UV light to photo-dissociate a silicon-carbon bond in linear silicone or linear fluorosilicone molecules contained in the coating fluid, and allowing them to form a new covalent bond to the surface of the solid substrate,
thus providing a solid substrate having at least one surface that carries a coating comprising a layer of linear silicone or linear fluorosilicone molecules covalently grafted to the surface and further comprising linear silicone or linear fluorosilicone molecules adhering non-covalently to the layer of the grafted linear silicone or linear fluorosilicone molecules.

**2.** The process in accordance with claim 1, which further comprises, subsequent to step c), a step of removing excess coating fluid from the solid substrate.

**3.** The process in accordance with claim 1 or 2, wherein the surface to be coated comprises or consists of a material selected from metal, metal oxide, glass, ceramic, or silicon.

**4.** The process in accordance with any of claims 1 to 3, wherein the surface to be coated comprises or consists of a material selected from steel, including stainless, martensitic or carbon steel, titanium, a titanium alloy, cobalt, magnesium, a magnesium alloy, glass, and porcelain.

**5.** The process in accordance with any of claims 1 to 4, wherein the surface to be coated is free of a metal-oxide photocatalyst.

**6.** The process in accordance with any of claims 1 to 5, wherein the solid substrate is selected from a medical device or a part thereof, a glass lens, marine equipment, a solar panel or a part thereof, and sanitary ware.

**7.** The process in accordance with any of claims 1 to 6, wherein the coating fluid is a neat silicone fluid or a neat fluorosilicone fluid.

**8.** The process in accordance with any of claims 1 to 7, wherein the linear silicone or linear fluorosilicone molecules contain a trimethylsiloxy group as an end group at one or both ends, more preferably as an end group at both ends.

**9.** The process in accordance with any of claims 1 to 8, wherein the coating fluid comprises or consists of polydimethylsiloxane.

**10.** The process in accordance with any of claims 1 to 9, wherein the irradiation of the coating fluid by UV-light in step c) is carried out for a period of 1 min or more, preferably 5 min or more.

**11.** The process in accordance with any of claims 1 to 10, wherein the linear silicone or linear fluorosilicone molecules contain a trimethylsiloxy group as an end group at one or both ends, preferably as an end group at both ends, and wherein the irradiation by UV-light photo-dissociates a silicon-carbon bond in a trimethylsiloxy end group of the linear silicone or linear fluorosilicone molecules.

**12.** The process in accordance with any of claims 1 to 11, wherein the irradiating UV light includes light with a wavelength of 321 nm or less.

**13.** A silicone or fluorosilicone-coated solid substrate having at least one surface that carries a coating comprising a layer of linear silicone or linear fluorosilicone molecules covalently grafted to the surface, and optionally further comprising linear silicone or linear fluorosilicone molecules adhering non-covalently to the layer of the grafted linear silicone or linear fluorosilicone molecules, which is obtainable by the process in accordance with any of claims 1 to 12.

**14.** The silicone or fluorosilicone-coated solid substrate in accordance with claim 13, wherein the thickness of the layer of the grafted linear silicone or linear fluorosilicone molecules, in the absence of linear silicone or linear fluorosilicone molecules adhering non-covalently to the layer of the grafted linear silicone or linear fluorosilicone molecules, is in the range of 3 to 100 nm, preferably at 5 to 20 nm.

**15.** Use of a silicone- or fluorosilicone-coated solid substrate in accordance with claim 13 or 14 in an application requiring one or more characteristics selected from corrosion resistance, reduced surface friction, capability for self-cleaning, anti-chemo-fouling, anti-bacterial, anti-marine fouling, and blood-repellency.

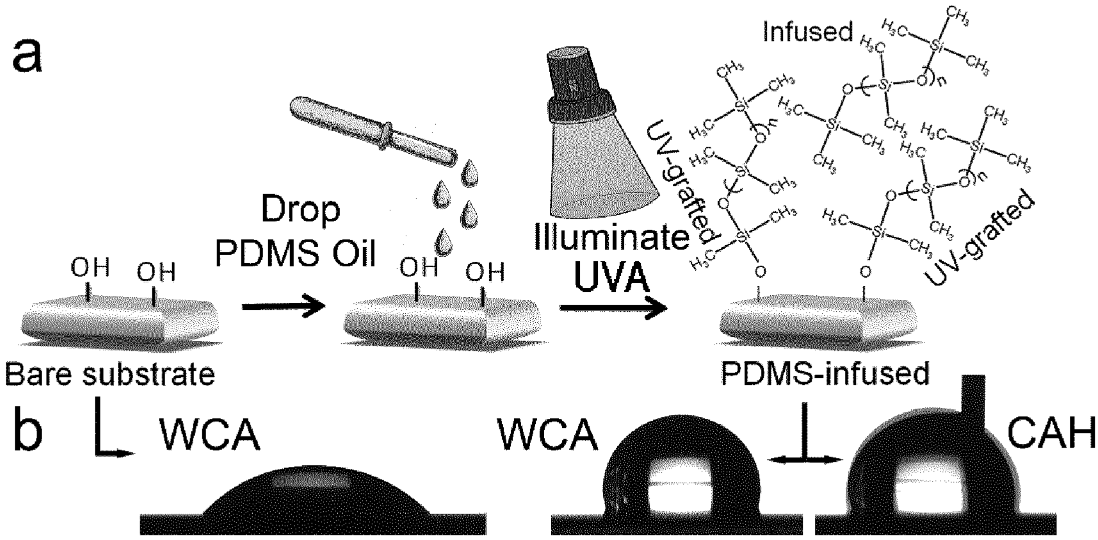
a
Drop
PDMS Oil
Illuminate
UVA
UV-grafted
Infused
UV-grafted
OH OH
OH OH
Bare substrate
PDMS-infused
b
WCA
WCA
CAH

Fig. 1a-b

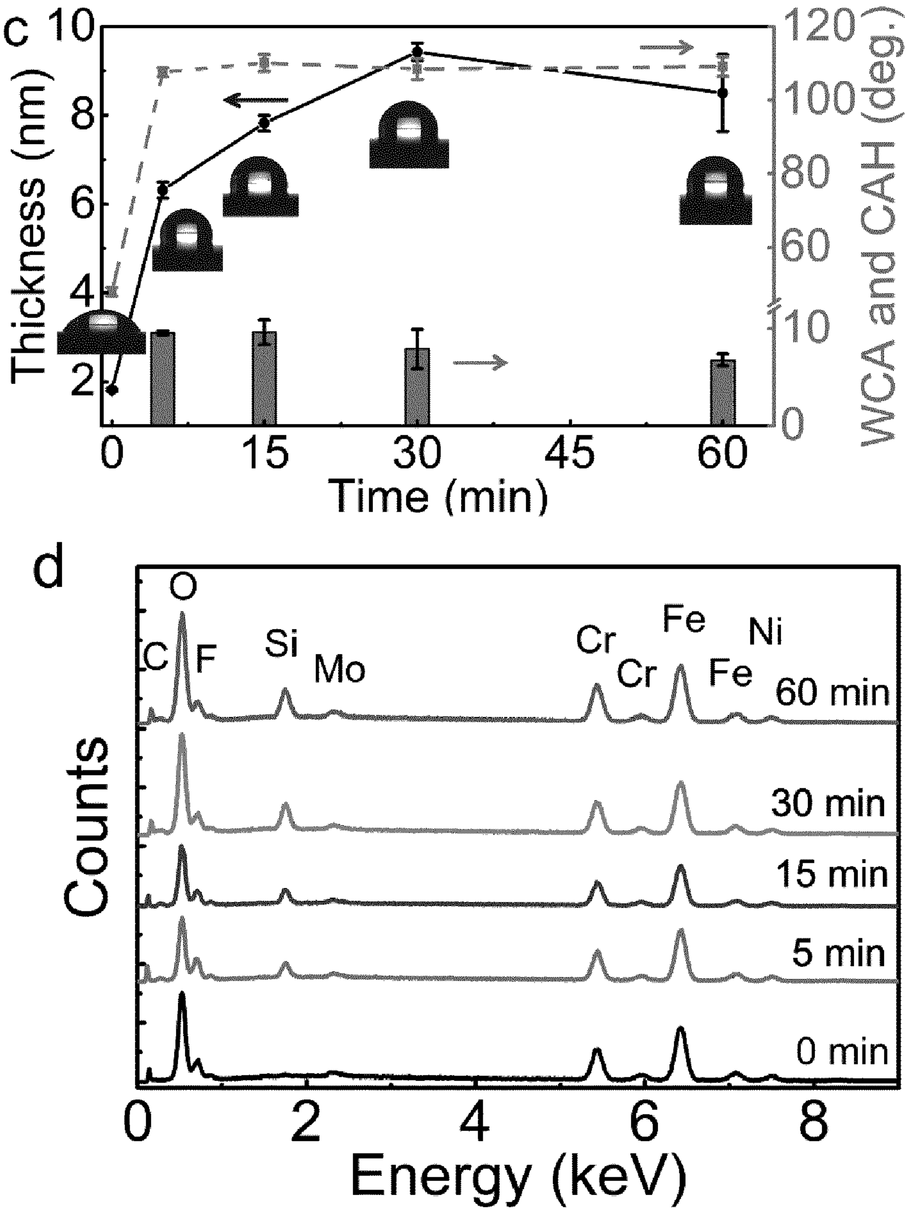
c
Thickness (nm)
WCA and CAH (deg.)
Time (min)
0
15
30
45
60
2
4
6
8
10
0
10
60
80
100
120
d
Counts
Energy (keV)
C
O
F
Si
Mo
Cr
Cr
Fe
Fe
Ni
60 min
30 min
15 min
5 min
0 min
0
2
4
6
8

Fi

Fig. 1c-d

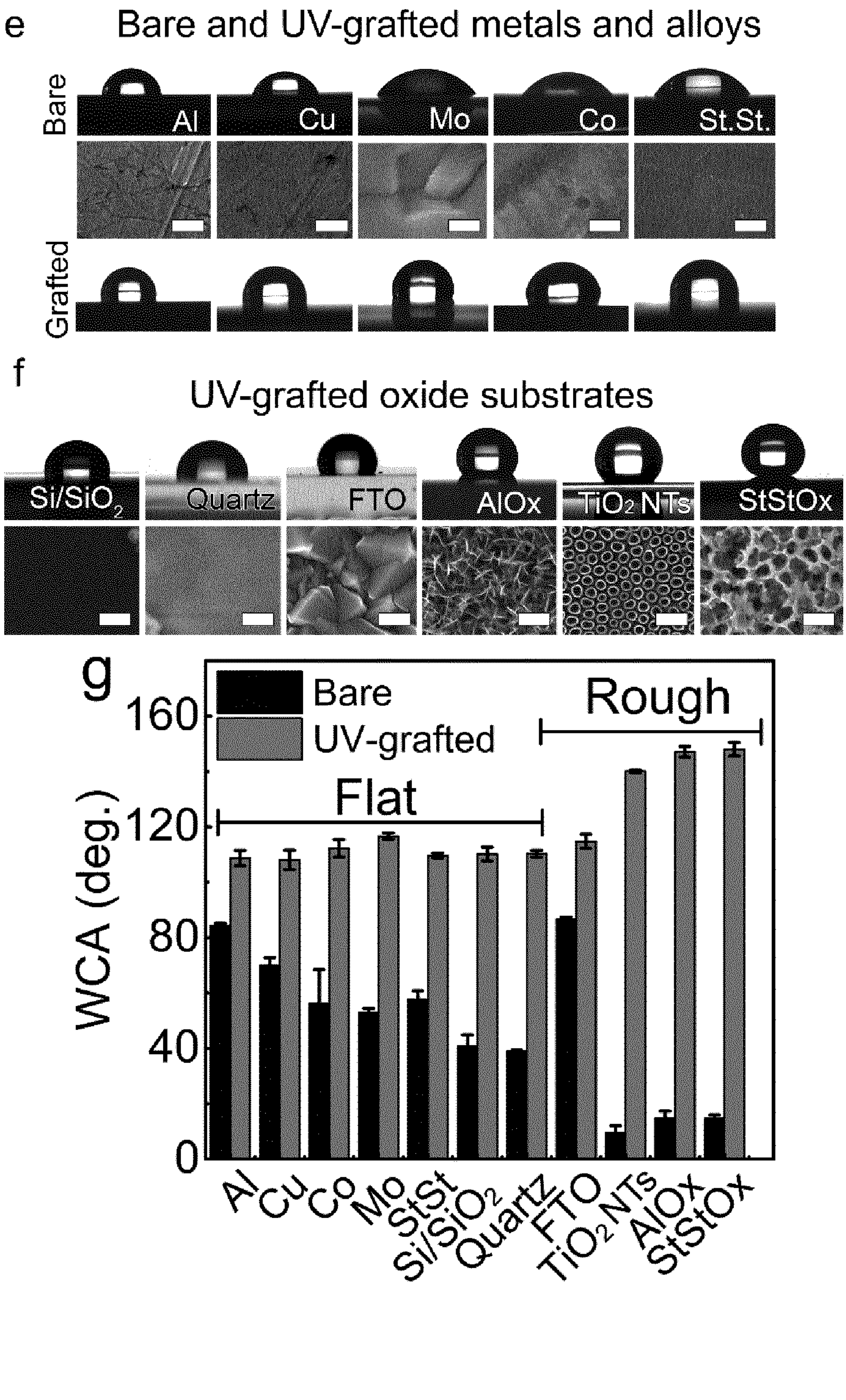
e
Bare and UV-grafted metals and alloys
Bare
Al
Cu
Mo
Co
St.St.
Grafted
f
UV-grafted oxide substrates
Si/SiO2
Quartz
FTO
AlOx
TiO2 NTs
StStOx
g
Bare
UV-grafted
Rough
Flat
WCA (deg.)
160
120
80
40
0
Al
Cu
Co
Mo
StSt
Si/SiO2
Quartz
FTO
TiO2 NTs
AlOx
StStOx

Fig. 1e-g

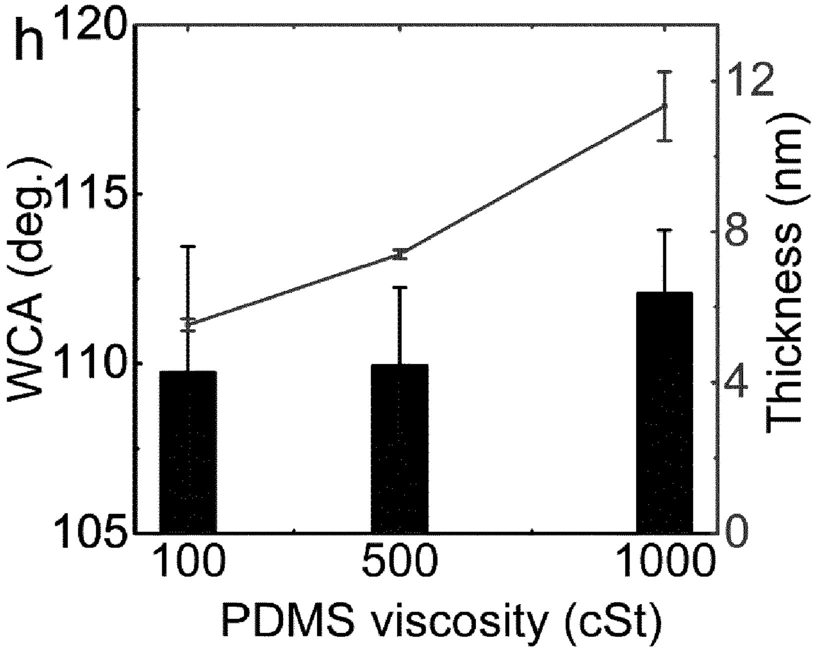


Fig. 1h

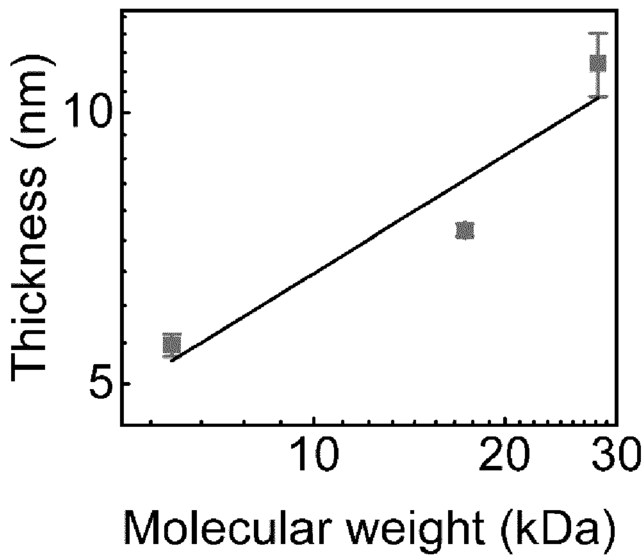


Figure 2

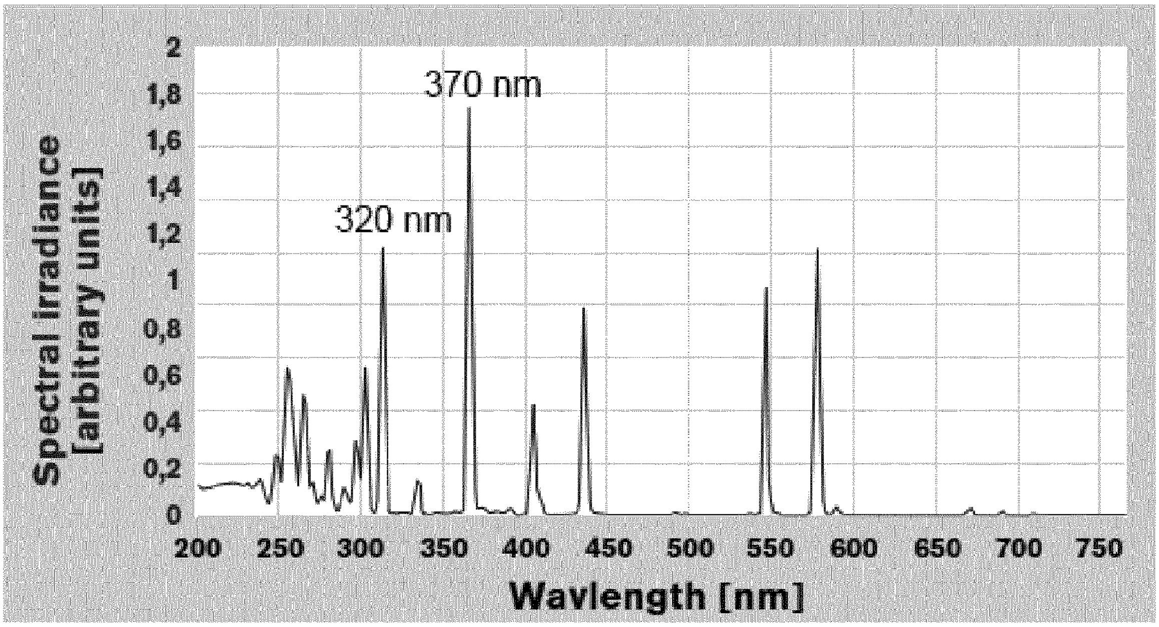
370 nm
320 nm
Spectral Irradiance [arbitrary units]
2
1,8
1,6
1,4
1,2
1
0,8
0,6
0,4
0,2
0
200 250 300 350 400 450 500 550 600 650 700 750
Wavlength [nm]

Fig. 3

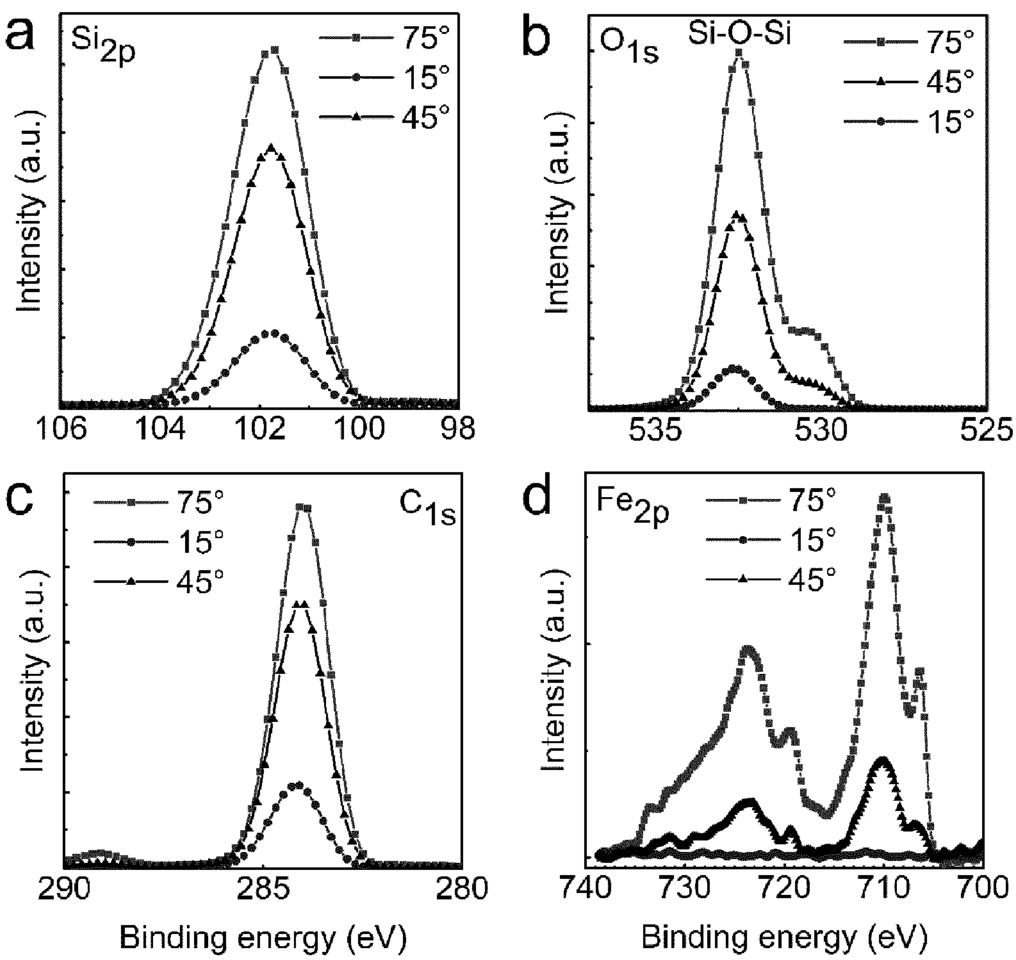
a
Si2p
75°
15°
45°
Intensity (a.u.)
106 104 102 100 98
b
O1s
Si-O-Si
75°
45°
15°
Intensity (a.u.)
535 530 525
c
C1s
75°
15°
45°
Intensity (a.u.)
290 285 280
Binding energy (eV)
d
Fe2p
75°
15°
45°
Intensity (a.u.)
740 730 720 710 700
Binding energy (eV)

Fig. 4a-d

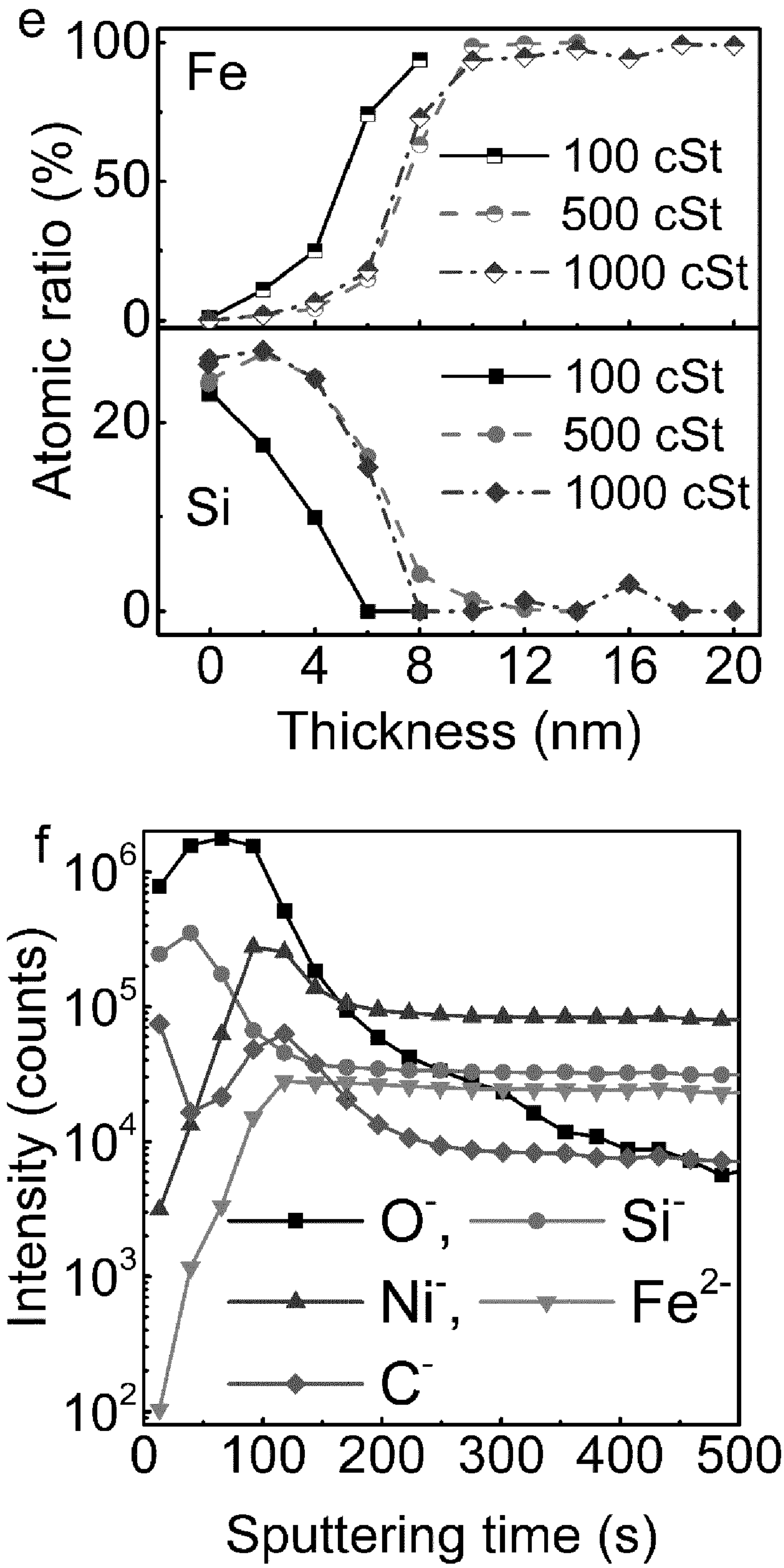
e
Fe
Si
Atomic ratio (%)
100
50
0
20
0
100 cSt
500 cSt
1000 cSt
100 cSt
500 cSt
1000 cSt
0
4
8
12
16
20
Thickness (nm)
f
Intensity (counts)
$10^6$
$10^5$
$10^4$
$10^3$
$10^2$
$O^-$, $Si^-$
$Ni^-$, $Fe^{2-}$
$C^-$
0
100
200
300
400
500
Sputtering time (s)

Figure 4e-f

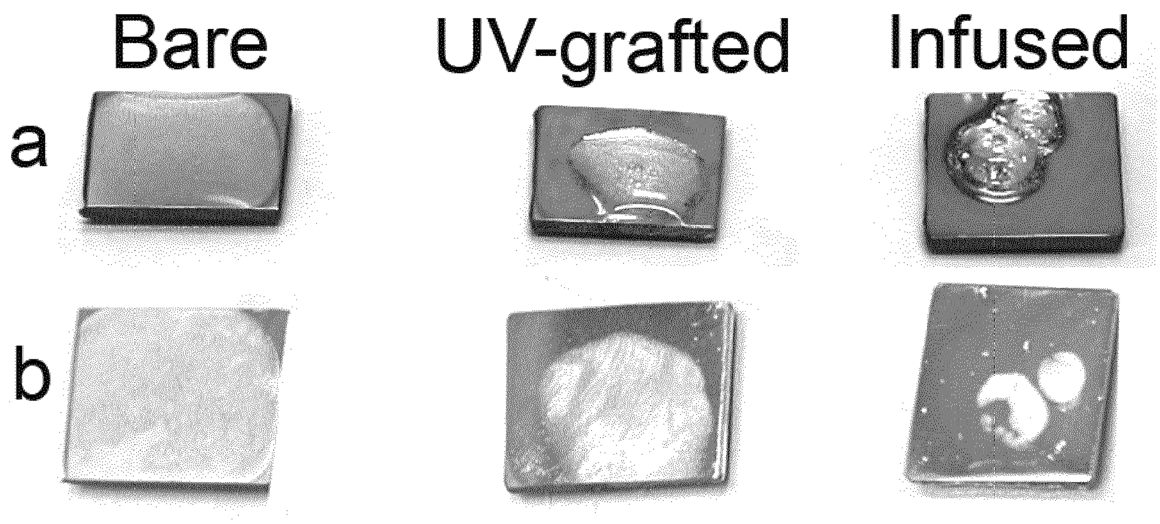


Figure 5

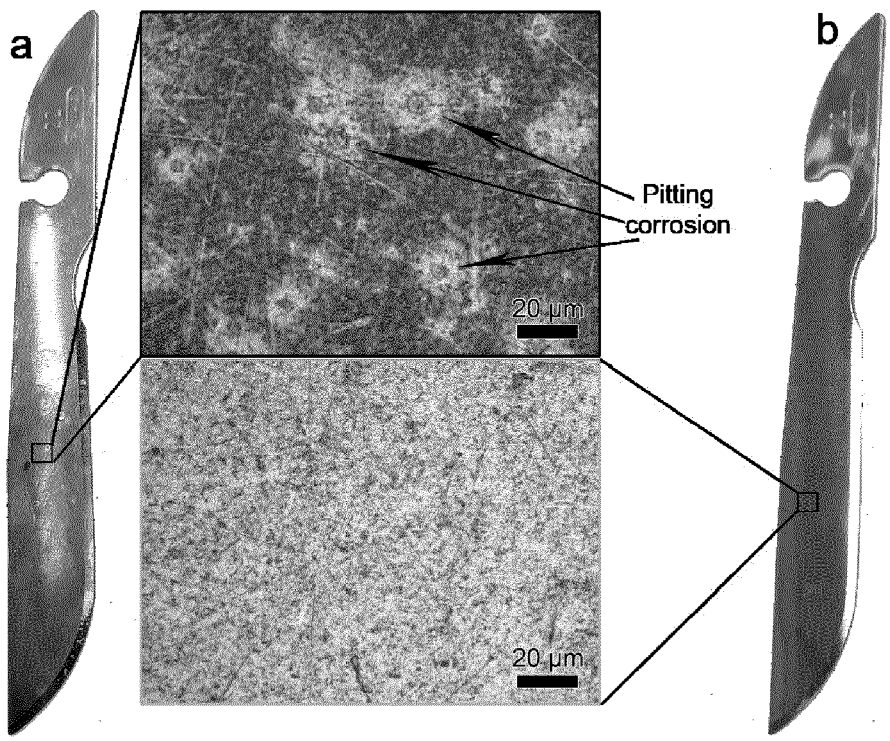


Figure 6

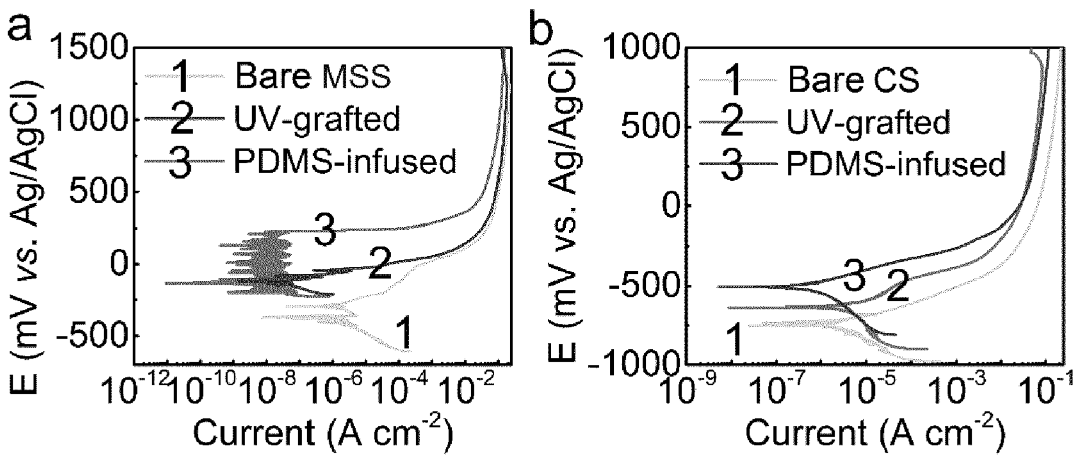


Fig. 7a-b

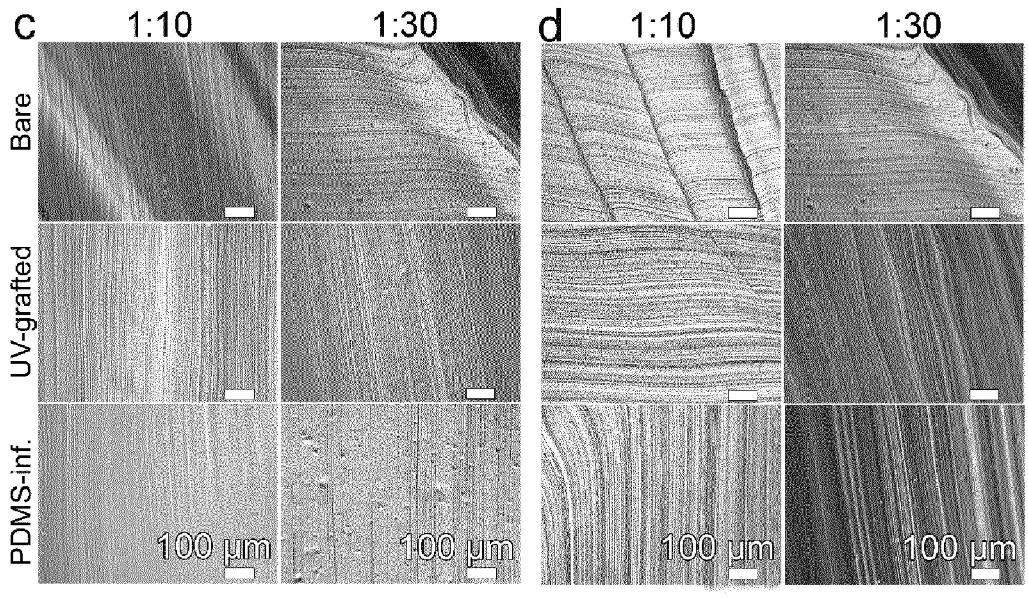


Fig. 7c-d

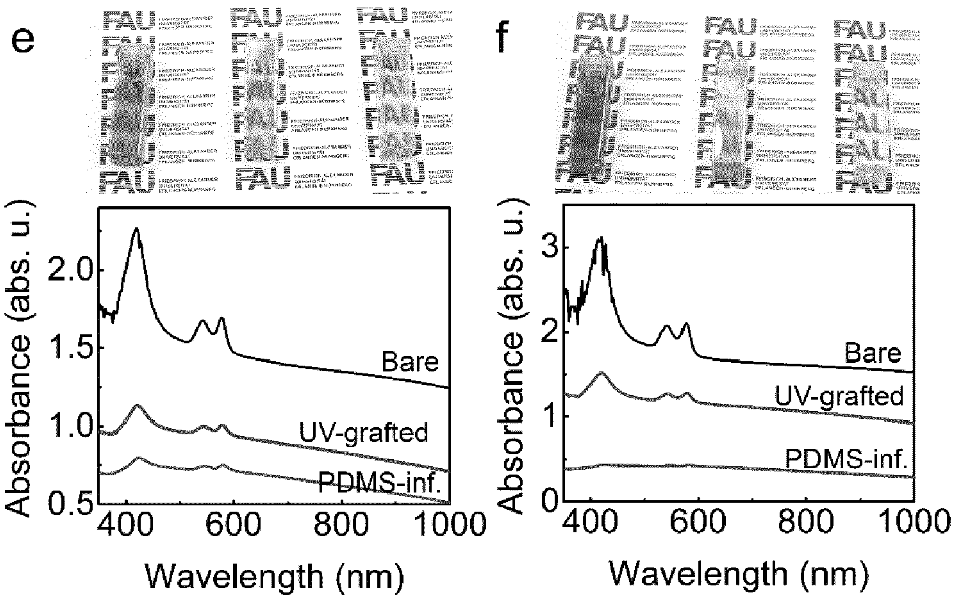
e
f
Absorbance (abs. u.)
2.0
1.5
1.0
0.5
3
2
1
0
Bare
UV-grafted
PDMS-inf.
400
600
800
1000
Wavelength (nm)

Fig. 7e-f

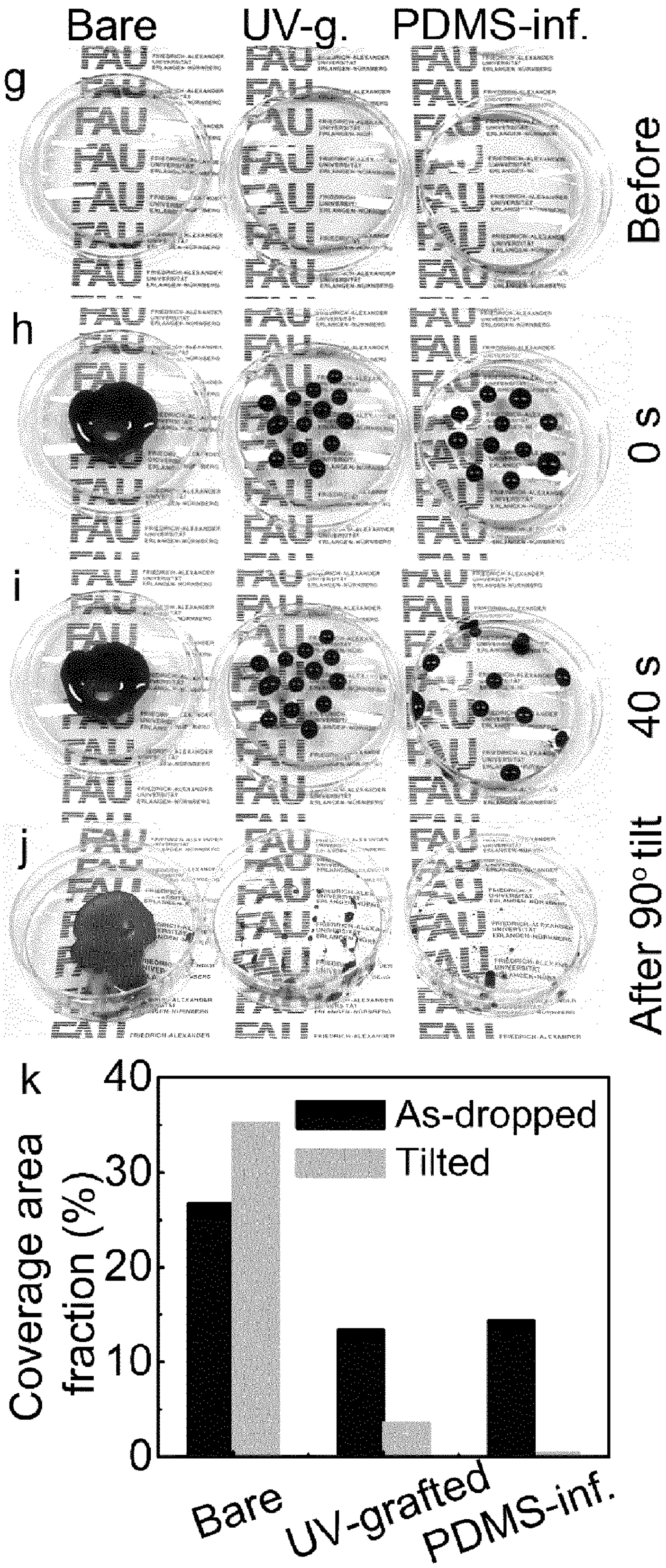
Bare
UV-g.
PDMS-inf.
g
Before
h
0 s
i
40 s
j
After 90° tilt
k
Coverage area fraction (%)
40
30
20
10
0
As-dropped
Tilted
Bare
UV-grafted
PDMS-inf.

Figure 7g-k

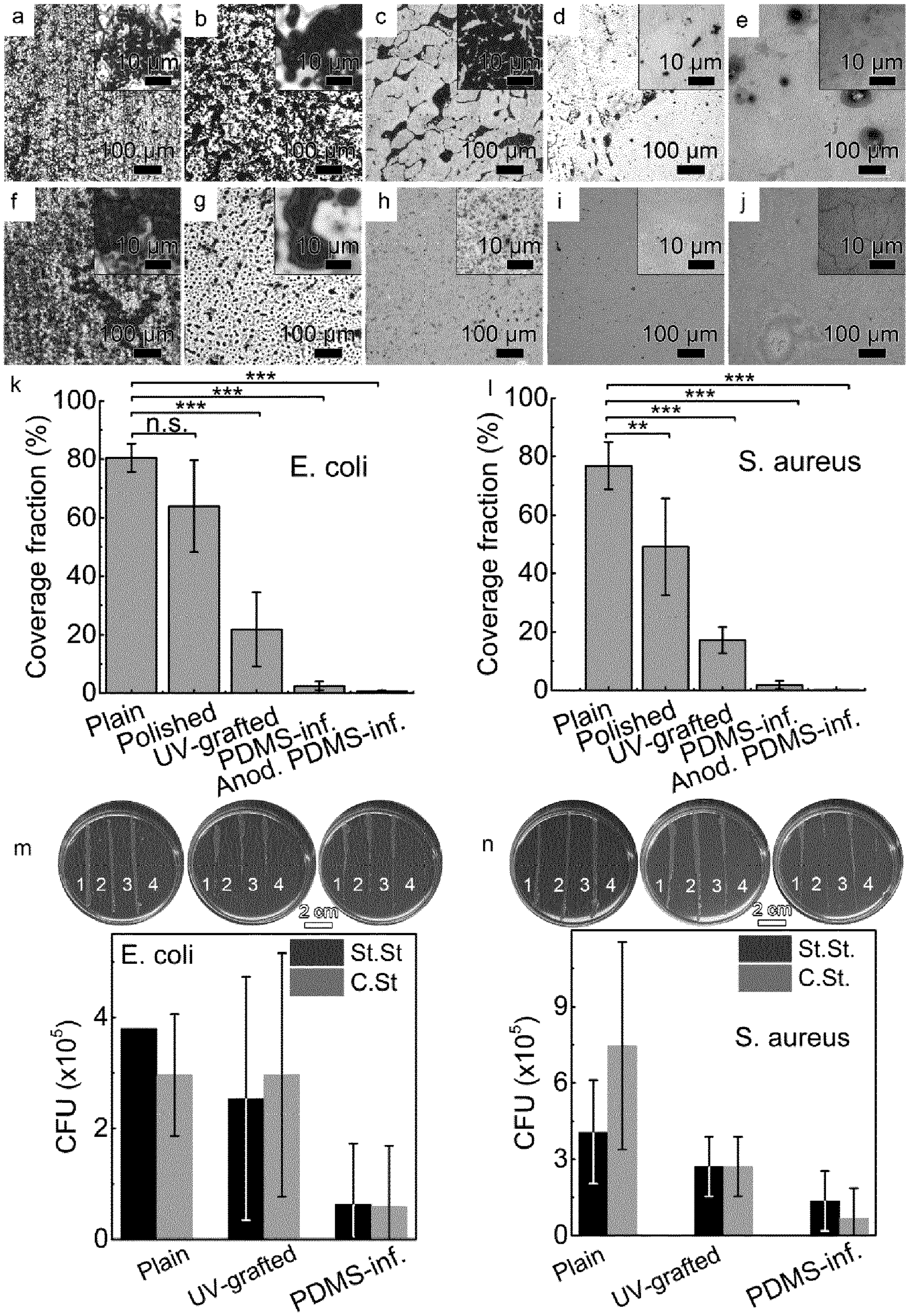
a
10 µm
100 µm
b
10 µm
100 µm
c
10 µm
100 µm
d
10 µm
100 µm
e
10 µm
100 µm
f
10 µm
100 µm
g
10 µm
100 µm
h
10 µm
100 µm
i
10 µm
100 µm
j
10 µm
100 µm
k
***
***
***
n.s.
Coverage fraction (%)
E. coli
Plain
Polished
UV-grafted
PDMS-inf.
Anod. PDMS-inf.
l
***
***
***
**
Coverage fraction (%)
S. aureus
Plain
Polished
UV-grafted
PDMS-inf.
Anod. PDMS-inf.
m
1 2 3 4
2 cm
n
1 2 3 4
2 cm
E. coli
St.St
C.St
CFU (x10^5)
Plain
UV-grafted
PDMS-inf.
St.St.
C.St.
S. aureus
CFU (x10^5)
Plain
UV-grafted
PDMS-inf.

Figure 8

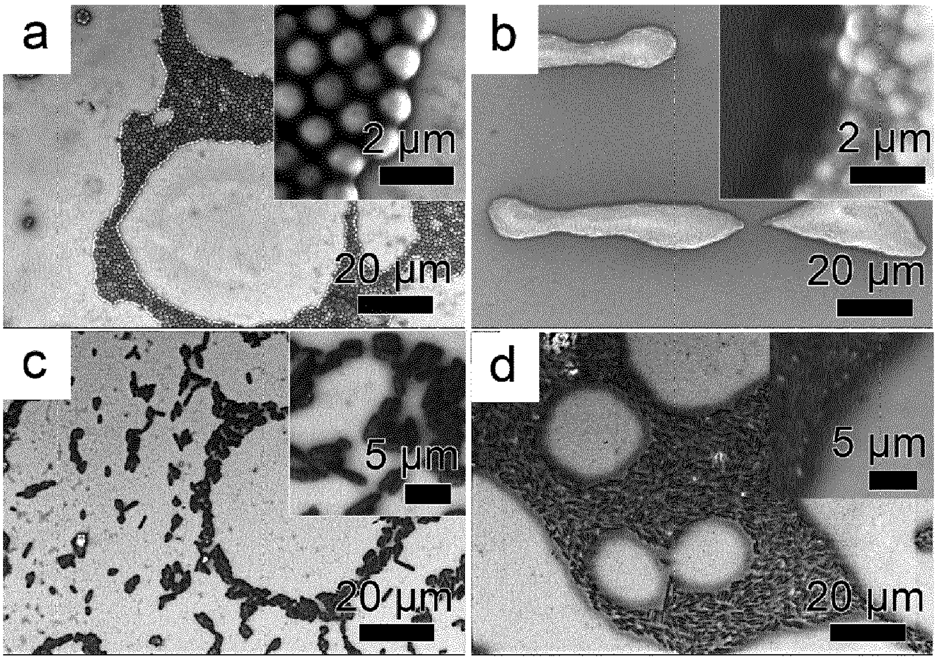


Figure 9

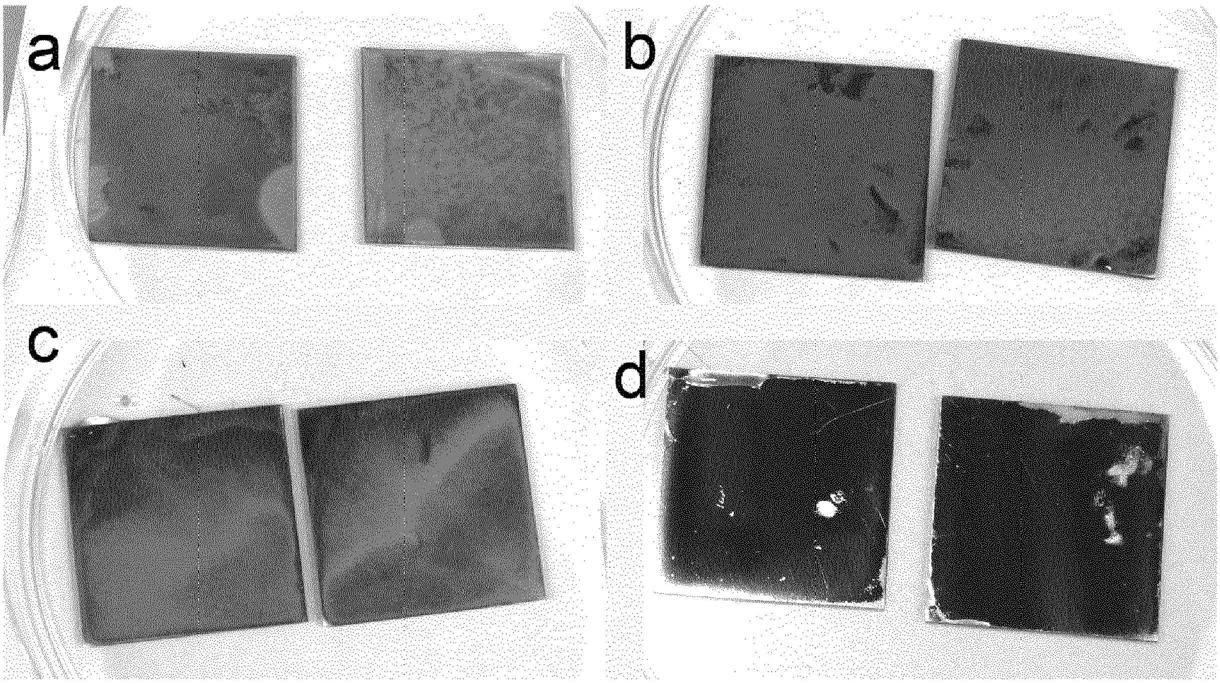


Figure 10

Europäisches Patentamt
European Patent Office
Office européen des brevets

# EUROPEAN SEARCH REPORT

Application Number
EP 21 16 6558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/137154 A1 (MAX-PLANCK-GESELLSCHAFT ZUR FÖRDERUNG DER WSS E V [DE]) 17 August 2017 (2017-08-17) * claims 1-6,8,10-13,15-17 * * page 8, paragraph 2-6 * * page 9, paragraph 5-6 * * figures; examples * ----- | 1-4,6-15 | INV. C09D183/04 C09D183/08 C08J3/28 C09D5/08 C09D5/16 B05D3/06 B05D5/08 B05D7/14 B08B17/02 B82Y30/00 B82Y40/00 ADD. G02B1/18 C03C17/30 C03C23/00 A61L33/06 |
| X | CN 110 818 278 A (UNIV SUN YAT SEN) 21 February 2020 (2020-02-21) | 13,15 | |
| A | * claims 1,4-6,8,9 * * figures 1-4; examples 1,6-9,11,12; tables 1-2 * ----- | 1-9,14 | |
| X,D | CHEN LIWEI ET AL: "One-Step Fabrication of Universal Slippery Lubricated Surfaces", ADVANCED MATERIALS INTERFACES, vol. 7, no. 18, 2000305, 29 July 2020 (2020-07-29), pages 1-9, XP055853339, DE ISSN: 2196-7350, DOI: 10.1002/admi.202000305 Retrieved from the Internet: URL:https://onlinelibrary.wiley.com/doi/full-xml/10.1002/admi.202000305> | 13-15 | TECHNICAL FIELDS SEARCHED (IPC) C09D C08L C08J B05D B03D B08B G02B C03C A61B A61L C10M C08G |
| A | * page 7, paragraph 2 - page 8, paragraph 4 * * abstract; figures 1-3,5-6 * ----- -/-- | 1-9 | |

1 The present search report has been drawn up for all claims

| Place of search | Date of completion of the search | Examiner |
|---|---|---|
| Munich | 4 November 2021 | Hollender, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 16 6558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br><br><br><br><br><br><br><br><br><br><br><br>A | TEISALA HANNU ET AL: "Grafting Silicone at Room Temperature - a Transparent, Scratch-resistant Nonstick Molecular Coating", LANGMUIR, vol. 36, no. 16, 28 April 2020 (2020-04-28), pages 4416-4431, XP055853303, US ISSN: 0743-7463, DOI: 10.1021/acs.langmuir.9b03223 Retrieved from the Internet: URL:https://pubs.acs.org/doi/pdf/10.1021/acs.langmuir.9b03223><br>* abstract; figures 1-4; tables 1-5 *<br>----- | 13-15<br><br><br><br><br><br><br><br><br><br><br><br><br><br>1-9 | C08L83/04<br>A61B1/313<br>A61B1/12<br>A61B17/3211<br>C10M107/50 |
| | | | TECHNICAL FIELDS SEARCHED (IPC) |

The present search report has been drawn up for all claims

| Place of search | Date of completion of the search | Examiner |
|---|---|---|
| Munich | 4 November 2021 | Hollender, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO.

EP 21 16 6558

This annex lists the patent family members relating to the patent documents cited in the above-mentioned European search report. The members are as contained in the European Patent Office EDP file on
The European Patent Office is in no way liable for these particulars which are merely given for the purpose of information.

04-11-2021

| Patent document cited in search report | Publication date | Patent family member(s) | Publication date |
|---|---|---|---|
| WO 2017137154 A1 | 17-08-2017 | EP 3205399 A1<br>WO 2017137154 A1 | 16-08-2017<br>17-08-2017 |
| CN 110818278 A | 21-02-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012100100 A2
- US 20190136070 A


### Non-patent literature cited in the description


- **G. B. HWANG ; K. PAGE ; A. PATIR ; S. P. NAIR ; E. ALLAN ; I. P. PARKIN.** *ACS Nano,* 2018, vol. 12, 6050
- **A. LAFUMA ; D. QUÉRÉ.** *EPL (Europhysics Letters),* 2011, vol. 96, 56001
- **T.-S. WONG ; S. H. KANG ; S. K. Y. TANG ; E. J. SMYTHE ; B. D. HATTON ; A. GRINTHAL ; J. AIZENBERG.** *Nature,* 2011, vol. 477, 443
- **R. DENG ; T. SHEN ; H. CHEN ; J. LU ; H.-C. YANG ; W. LI.** *J. Mat. Chem. A,* 2020, vol. 8, 7536
- **J. W. KRUMPFER ; T. J. MCCARTHY.** *Langmuir,* 2011, vol. 27, 11514
- **S. T. MILNER ; T. A. WITTEN ; M. E. CATES.** *Macromolecules,* 1988, vol. 21, 2610
- **C.-H. XUE ; X. BAI ; S.-T. JIA.** *Sci. Rep.,* 2016, vol. 6, 27262
- **L. CHEN ; S. PARK ; J. YOO ; H. HWANG ; H. KIM ; J. LEE ; J. HONG ; S. WOOH.** *Adv. Mater. Interfaces,* 2020, vol. 7, 2000305
- **C. ZHANG ; Y. XIA ; H. ZHANG ; N. S. ZACHARIA.** *ACS Appl. Mater. Interfaces,* 2018, vol. 10, 5892
- **H. TEISALA ; P. BAUMLI ; S. A. L. WEBER ; D. VOLLMER ; H.-J. BUTT.** *Langmuir,* 2020, vol. 36, 4416
- **S. WOOH ; N. ENCINAS ; D. VOLLMER ; H.-J. BUTT.** *Adv. Mater.,* 2017, vol. 29, 1604637
- **J. WANG ; L. WANG ; N. SUN ; R. TIERNEY ; H. LI ; M. CORSETTI ; L. WILLIAMS ; P. K. WONG ; T.-S. WONG.** *Nat. Sustain.,* 2019, vol. 2, 1097
- **C. YANG ; F. WANG ; W. LI ; J. OU ; C. LI ; A. AMIRFAZLI.** *Appl. Phys. A: Mater. Sci. Process.,* 2015, vol. 122, 1
- **L. PRADO ; E. ANASTASIOU ; S. VIRTANEN.** *J. Electrochem. Soc.,* 2021, vol. 168, 021507
- **A. B. TESLER ; M. ALTOMARE ; P. SCHMUKI.** *ACS Appl. Nano Mater.,* 2020, vol. 3, 10646
- **A. B. TESLER ; T. SANNOMIYA ; A. VASKEVICH ; E. SABATANI ; I. RUBINSTEIN.** *Adv. Opt. Mater.,* 2018, vol. 6, 1800599
- **J. COLTER STEWART ; M. N. SHELLEY ; N. R. SCHWARTZ ; S. K. KING ; D. W. BOYCE ; J. W. ERIKSON ; D. D. ALLRED ; J. S. COLTON.** *Opt. Mater. Express,* 2019, vol. 9, 4677
- **J. A. MYERS ; B. S. CURTIS ; W. R. CURTIS.** *BMC Biophysics,* 2013, vol. 6 (4
- **Y. ZHAO ; D. G. TRUHLAR.** *Theor. Chem. Acc.,* 2008, vol. 120, 215
- **F. WEIGEND ; R. AHLRICHS.** *Phys. Chem. Chem. Phys.,* 2005, vol. 7, 3297
- **F. WEIGEND.** *Phys. Chem. Chem. Phys.,* 2006, vol. 8, 1057
- **J. LIU ; L. YE ; S. WOOH ; M. KAPPL ; W. STEFFEN ; H.-J. BUTT.** *ACS Appl. Mater. Interfaces,* 2019, vol. 11, 27422
- **B. SCHNYDER ; T. LIPPERT ; R. KÖTZ ; A. WOKAUN ; V.-M. GRAUBNER ; O. NUYKEN.** *Surf. Sci.,* 2003, vol. 532-535, 1067
- **M. BRINKMANN ; V. Z. H. CHAN ; E. L. THOMAS ; V. Y. LEE ; R. D. MILLER ; N. HADJICHRISTIDIS ; A. AVGEROPOULOS.** *Chem. Mater.,* 2001, vol. 13, 967
- **M. OUYANG ; R. J. MUISENER ; A. BOULARES ; J. T. KOBERSTEIN.** *J. Membr. Sci.,* 2000, vol. 177, 177
- **S. TARDIO ; M.-L. ABEL ; R. H. CARR ; J. E. CASTLE ; J. F. WATTS.** *J. Vac. Sci. Technol., A,* 2015, vol. 33, 05E122
- **A. KAUR ; P. CHAHAL ; T. HOGAN.** *IEEE Electron Device Lett.,* 2016, vol. 37, 142
- **A. J. PERTSIN ; M. M. GORELOVA ; V. Y. LEVIN ; L. I. MAKAROVA.** *J. Appl. Polym. Sci.,* 1992, vol. 45, 1195
- **A. SABATA ; W. J. VAN OOIJ ; H. K. YASUDA.** *Surf. Interface Anal.,* 1993, vol. 20, 845
- **Y. HASHIMOTO ; T. YAMAMOTO.** *Appl. Surf. Sci.,* 2017, vol. 419, 319
- **D. DWIVEDI ; K. LEPKOVÁ ; T. BECKER.** *RSC Adv.,* 2017, vol. 7, 4580
- **A. B. TESLER ; P. KIM ; S. KOLLE ; C. HOWELL ; O. AHANOTU ; J. AIZENBERG.** *Nat. Commun.,* 2015, vol. 6, 8649
- **E. K. HANSON ; J. BALLANTYNE.** *PLoS One,* 2010, vol. 5, e12830

- **G. H. BALLANTYNE.** *Surg. Laparosc. Endosc. Percutan. Tech.,* 2002, vol. 12, 1
- **T. OKAMOTO ; T. OHNISHI ; H. KAWAHIRA ; O. DERGACHYAVA ; P. JANNIN ; H. HANEISHI.** *Signal Image Video P.,* 2019, vol. 13, 405
- **N. VOGEL ; R. A. BELISLE ; B. HATTON ; T.-S. WONG ; J. AIZENBERG.** *Nat. Commun.,* 2013, vol. 4, 2176
- **S. SUNNY ; G. CHENG ; D. DANIEL ; P. LO ; S. OCHOA ; C. HOWELL ; N. VOGEL ; A. MAJID ; J. AIZENBERG.** *Proc. Natl. Acad. Sci. U. S. A.,* 2016, vol. 113, 11676
- **Y. KOVALENKO ; I. SOTIRI ; J. V. I. TIMONEN ; J. C. OVERTON ; G. HOLMES ; J. AIZENBERG ; C. HOWELL.** *Adv. Healthc. Mater.,* 2017, vol. 6, 1600948
- **J. A. MYERS ; B. S. CURTIS ; W. R. CURTIS.** *BMC Biophysics,* 2013, vol. 6, 4